(19) Europäisches Patentamt
European Patent Office
Office européen des brevets

(11) EP 2 760 059 A1

(12) EUROPEAN PATENT APPLICATION
published in accordance with Art. 153(4) EPC

(43) Date of publication:
30.07.2014 Bulletin 2014/31

(21) Application number: 12834348.0

(22) Date of filing: 23.07.2012

(51) Int Cl.:
*H01L 51/50* (2006.01) *C07D 519/00* (2006.01)
*C09K 11/06* (2006.01) *C07F 15/00* (2006.01)

(86) International application number:
PCT/JP2012/068571

(87) International publication number:
WO 2013/042446 (28.03.2013 Gazette 2013/13)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 21.09.2011 JP 2011206277

(71) Applicant: Konica Minolta, Inc.
Tokyo 100-7015 (JP)

(72) Inventors:
- OIKAWA, Kazuhiro
Hachioji-shi
Tokyo 192-8505 (JP)
- HIYAMA, Kunimasa
Hachioji-shi
Tokyo 192-8505 (JP)

(74) Representative: Green, Mark Charles
Urquhart-Dykes & Lord LLP
The Podium
1 Eversholt Street
London NW1 2DN (GB)

# (54) ORGANIC ELECTROLUMINESCENT ELEMENT, DISPLAY DEVICE, AND ILLUMINATION DEVICE

(57) This organic electroluminescent element has, on a substrate (1), a positive electrode (2) and a negative electrode (8) that are paired electrodes, and at least one light-emitting layer (5). The light-emitting layer (5) contains at least one of each of a dopant and a host. The light-emitting layer (5) is formed by means of a coating method using a coating liquid comprising at least one solvent and has at least one peak in the distribution curve that is of the number frequency distribution of multimolecular aggregates formed from the dopant molecule and/or the host molecule and that is obtained from x-ray small-angle scattering. The smallest particle size value of the particle size values in the peak top is no greater than 4 nm.

*FIG.1*

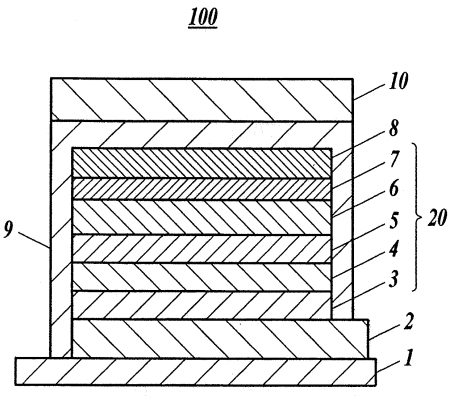


EP 2 760 059 A1

## Description

Technical Field

The present invention relates to an organic electroluminescent element, and specifically relates to an organic electroluminescent element that is excellent in a life span of light emission and a display device and an illumination device that use the organic electroluminescent element(s).

Background Art

In recent years, an organic electroluminescent element employing an organic compound(s) (hereinafter also referred to as an "organic EL element" arbitrarily) has been highly expected for its applications to, for example, a large-sized full-colored display element of solid-state light emission available at a low price and to writing light source arrays. Thus, research and development of such an organic electroluminescent element have been actively conducted.

An organic EL element is a thin all-solid-state element composed of a pair of an anode and cathode formed on/over a layer and an organic functional layer(s) provided between the anode and the cathode, and the organic functional layer(s) which may consists of a single or multiple layers contains an organic light-emitting compound(s) and has a thickness of only about 0.1 μm.

It is known that when a relatively low voltage of about 2 to 20 V is applied to such an organic EL element, electrons are injected from a cathode to an organic compound layer, and electron holes are injected from an anode to the organic compound layer; then these electrons and electron holes are recombined with each other in a light-emitting layer, and energy is released as light upon return of an electron energy level from a conduction band to a valence bond. This technique is expected for use in future flat displays and illumination devices.

In addition, an organic EL element utilizing phosphorescence emission, which has been recently found, can achieve efficiency of light emission of about four times larger in principle than that of a conventional element utilizing fluorescence emission. Thus, research and development of layer configurations of organic functional layers and electrodes of a light-emitting element utilizing phosphorescence emission have been extensively conducted all over the world, as well as developments of its materials. In particular, as one of measures for preventing global warming, an organic EL element utilizing phosphorescence emission has begun to be considered to be applied to illumination devices, which currently consumes large part of energy that human consumes. Hence, an organic EL element utilizing phosphorescence emission is extensively studied for improving its efficiency and for decreasing costs for practical realization of a white light-emitting panel, which can be an alternative for conventional illumination devices.

Such organic EL elements can be produced by vacuum deposition methods and wet processes (application methods). In recent years, methods by wet processes (spin coating, casting, ink jetting, spraying, printing, slot coating) have attracted attention because they do not need vacuum processes and achieve easy continuous production and high production speed.

An application-type organic EL element is typically exemplified by an application-type organic EL element using a polymer material(s) in its light-emitting layer(s) (see Patent Document 1, for example).

A coating-type polymer organic EL element is, however, of concern about performance and production stability, because it is difficult to purify its materials and control the distribution of their molecular weights.

Given this situation, an application-type low molecular organic EL element has been of interest in recent years.

An application-type organic EL element, which is produced using an application method(s), is disclosed (see Patent Document 2, for example), and a low molecular organic EL element excellent in efficiency of light emission, luminance and a half life is provided. However, an application-type low molecular organic EL element has insufficient properties to be used in an illumination device, and thus needs to achieve lower driving voltage and a longer life span.

When fine crystals are contained, resistance increases due to scattering of electron transfer caused by grain boundaries. Further, in a long driving of an organic electroluminescent element containing fine crystals, the fine crystals increase their sizes due to generated Joule heat, and resistance against transfer of electrons and holes increase with time, resulted in a rise in driving voltage and a decrease in its life span. Driving voltage rises and a life span shortens caused by generation of fine crystals as the sizes of the fine crystals increase.

To lower driving voltage in an application-type low molecular organic EL element, it is necessary to reduce the sizes of the contained fine crystals and reduce the number of the contained fine crystals.

There have been many prior art documents describing layer-forming application methods that can suppress inclusion of large-sized fine crystals.

For example, there is disclosed a method for forming thin amorphous layers using an ink-jetting application method.

There is disclosed a method for forming thin amorphous layers composed of organic materials selectively on depressed regions prepared by partition with dividing layers using an ink-jetting application method through acquiring

the optimum viscosity of ink in mixing two kinds of solvents and by increasing surface tension of the ink in a drying step and solubility limits of the above organic materials (see Patent Document 3, for example).

This method is effectively used for a general application method. In this document, however, formation of an amorphous layer is confirmed only by visual estimation. Thus, the possibility of generation of fine crystals that are too small to be visually observed cannot be dismissed.

In addition, there is disclosed a method for preventing crystallization of organic materials in heating by conducting drying with heat at a temperature 10°C or more higher than a glass transition temperature in an inert gas atmosphere after application of the organic materials in an inert gas atmosphere (see Patent Document 4, for example).

This method can prevent growth of crystals in drying with heat, but inevitably generates large fine crystals due to volatilization of solvents after the application to the drying with heat.

Further, there is a document disclosing a method for producing a deposition-type organic EL element including a thin layer(s) where no X-ray diffraction peak derived from fine crystals is found (see Patent Document 5, for example). Even so, there remains problems to be solved for achieving a method for producing an application-type low molecular organic EL element where no X-ray diffraction peak derived from fine crystals is found.

For solving the problems, there is disclosed an organic electroluminescent element where functional layers containing no fine crystal that can be observed by X-ray diffraction through increasing the speed of drying (see Patent Document 6).

In the above, a commercially-available apparatus such as a thin layer structure evaluator ATX-G manufactured by Rigaku Corporation is used for X-ray diffraction to determine that layers have no peak derived from fine crystals and to conclude that the layers therefore achieve high efficiency and a long life span.

However, in high-energy X-ray diffraction with high directivity, peaks derived from fine crystals are observed in such layers. This suggests that the molecules are not sufficiently dispersed in the layers but some of the molecules are aggregated to form domains (clusters formed by aggregation or voids). Therefore, the above disclosure are not sufficient for achieving an element having higher efficiency of light emission and a longer life span.

## Prior Art Document

### Patent Document

Patent Document 1: Japanese Patent Application Laid-Open Publication No. Hei6-33048

Patent Document 2: Japanese Patent Application Laid-Open Publication No. 2006-190759

Patent Document 3: Japanese Patent Application Laid-Open Publication No. 2006-66294

Patent Document 4: Japanese Patent Application Laid-Open Publication No. 2005-310639

Patent Document 5: Japanese Patent Application Laid-Open Publication No. 2005-276748

Patent Document 6: Japanese Patent Application Laid-Open Publication No. 2010-278287

## Summary of the Invention

### Problem to be Solved by the Invention

A main object of the present invention is therefore to provide an organic EL element having high efficiency of light emission and a long life span, and specifically, to provide an organic EL element where average sizes of a fine crystal(s) and an aggregate(s) present in a light-emitting layer(s) of the element are small and the molecules are sufficiently dispersed in layers. Another object of the present invention is to provide a display device and an illumination device that use the organic EL element(s).

### Means for Solving Problem

To solve the above problems, according to the present invention, there is provided an organic electroluminescent element including, on/over a substrate:

a pair of electrodes; and
at least one light-emitting layer, wherein
the light-emitting layer contains at least one kind of dopant and at least one kind of host,
the light-emitting layer is formed by an application method using an application liquid containing at least one kind of solvent,
a distribution curve of a number frequency distribution of multi-molecular aggregates of either or both of molecules of the dopant and the host in the light-emitting layer has at least one peak, which distribution curve is obtained from Small-angle X-ray scattering, and
a minimum particle size at peak top(s) of the peak(s) is 4 nm or less.

Effect of the Invention

According to the present invention, an organic electroluminescent element with high efficiency of light emission and a long life span can be provided by selecting a host material(s) and a phosphorescent dopant(s) in a light-emitting layer(s) and conditions for layer forming. Homogeneous layers can therefore be stably formed, and thus productivity is stabilized and a yield is increased. In addition, stability in storage is improved by increasing dispersibility of materials of the light-emitting layer(s), which is difficult to be achieved in common combinations.

Brief Description of Drawing

[Fig. 1] This is a schematic cross sectional view illustrating an example of a configuration of an organic electroluminescent element.
[Fig. 2] This is a diagram illustrating particle size distributions of Example Samples 1, 6 and 7 and Comparative Example Sample 11.

Embodiment for Carrying Out the Invention

Preferred embodiments of the present invention will now be described.

«Configuration of Organic EL Element»

As illustrated in Fig. 1, an organic electroluminescent element (hereinafter also referred to as an organic EL element) 100 includes a flexible supporting substrate 1. On the flexible supporting substrate 1, an anode 2 is formed; on/over the anode 2, organic functional layers 20 are formed; and on the organic functional layer 20, a cathode 8 is formed.
The organic functional layers 20 represent layers provided between the anode 2 and the cathode 8 and being constituents of the organic EL element 100.
The organic functional layers 20 include, for example, a hole-injecting layer 3, a hole-transporting layer 4, a light-emitting layer 5, an electron-transporting layer 6 and an electron-injecting layer 7. The organic functional layers 20 can also include a hole-blocking layer, an electron-blocking layer and the like.
The anode 2, the organic functional layers 20 and the cathode 8 on/over the flexible supporting substrate 1 are sealed with a flexible sealing member 10.
This layer configuration of the organic electroluminescent element 100 (see Fig. 1) is merely a preferable example, and the present invention is not limited thereto. The organic EL element 100 may have any of the following layer configurations, for example:

(i) flexible supporting substrate/anode/light-emitting layer/electron-transporting layer/cathode/heat-conducting layer/sealing adhesive/sealing member
(ii) flexible supporting substrate/anode/hole-transporting layer/light-emitting layer/electron-transporting layer/cathode/heat-conducting layer/sealing adhesive/sealing member
(iii) flexible supporting substrate/anode/hole-transporting layer/light-emitting layer/hole-blocking layer/electron-transporting layer/cathode/heat-conducting layer/sealing adhesive/sealing member
(iv) flexible supporting substrate/anode/hole-transporting layer/light-emitting layer/hole-blocking layer/electron-transporting layer/cathode buffer layer/cathode/heat-conducting layer/sealing adhesive/sealing member
(v) flexible supporting substrate/anode/anode buffer layer/hole-transporting layer/light-emitting layer/hole-blocking layer/electron-transporting layer/cathode buffer layer/cathode/heat-conducting layer/sealing adhesive/sealing member

(vi) glass support/anode/hole-injecting layer/light-emitting layer/electron-injecting layer/cathode/sealing member
(vii) glass support/anode/hole-injecting layer/hole-transporting layer/light-emitting layer/electron-injecting layer/cathode/sealing member
(viii) glass support/anode/hole-injecting layer/hole-transporting layer/light-emitting layer/electron-transporting layer/electron-injecting layer/cathode/sealing member

«Organic Functional Layers 20 of Organic EL Element»

Details of the organic functional layers as constituents of the organic EL element of the present invention will now be described.

(1) Injecting Layer: Hole-Injecting Layer 3, Electron-injecting Layer 7

In the organic EL element of the present invention, an injecting layer(s) may be provided as needed. The injecting layers are categorized into an electron-injecting layer and a hole-injecting layer. The injecting layer may be provided between the anode and the light-emitting layer or between the anode and the hole-transporting layer, or between the cathode and the light-emitting layer or between the cathode and the electron-transporting layer, as described above.

The injecting layer of the present invention is a layer provided between the electrode and the organic functional layer to lower driving voltage and improve luminance, and described in detail in Chapter 2 "Electrode Materials", Div. 2 Chapter 2 (pp. 123-166) of "Organic EL element and its frontier of industrialization" (published by NTS Corporation, November 30, 1998). The injecting layers are categorized into an electron-injecting layer and an electron hole-injecting layer.

Details of the hole-injecting layer are also described in, for example, Japanese Patent Application Laid-Open Publications Nos. Hei9-45479, Hei9-260062 and Hei8-288069. Examples of the hole-injecting materials that can be used in the hole-injecting layer include polymers and aniline copolymers that contain a triazole derivative(s), oxadiazole derivative(s), imidazole derivative(s), pyrazoline derivative(s), pyrazolone derivative(s), phenylenediamine derivative(s), arylamine derivative(s), amino-substituted chalcone derivative(s), oxazole derivatives(s), styrylanthracene derivative(s), fluorenone derivative(s), hydrazone derivative(s), stilbene derivative(s) and silazane derivative(s); polyarylalkane derivative(s); and electroconductive polymers. Polythiophene derivatives, polyaniline derivatives and polypyrrole derivatives are preferable, and polythiophene derivatives are more preferable.

Details of the electron-injecting layer are also described in, for example, Japanese Application Laid-Open Publications Nos. Hei6-325871, Hei9-17574 and Hei10-74586, and is specifically exemplified by a buffer layer composed of a metal as typified by strontium, aluminum and the like, a buffer layer composed of an alkali metal as typified by lithium fluoride, a buffer layer composed of an alkali earth metal as typified by magnesium fluoride, a buffer layer composed of an oxide as typified by aluminum oxide. In the present invention, the buffer layer (i.e., injecting layer) is preferably a very thin layer and is preferably composed of potassium fluoride or sodium fluoride. The thickness of the injecting layer is from 0.1 nm to 5 $\mu$m, preferably from 0.1 to 100 nm, more preferably from 0.5 to 10 nm, and most preferably 0.5 to 4 nm.

(2) Hole-Transporting Layer 4

A hole-transporting material of the hole-transporting layer may be a compound used in the hole-injecting layer. Preferably, the hole-transporting material is a porphyrin compound, aromatic tertiary amine compound or stylylamine compound, and more preferably an aromatic tertiary amine compound. Representative examples of the aromatic tertiary anime compounds and stylylamine compounds include N,N,N',N'-tetraphenyl-4,4'-diaminophenyl;
N,N'-diphenyl-N,N'-bis(3-methylphenyl)-[1,1'-biphenyl]-4,4'-diamine (TPD);
2,2-bis(4-di-p-tolylaminophenyl)propane; 1,1-bis(4-di-p-tolylaminophenyl)cyclohexane;
N,N,N',N'-tetra-p-tolyl-4,4'-diaminobiphenyl;
1,1-bis (4-di-p-tolylaminophenyl)-4-phenylcyclohexane;
bis(4-dimethylamino-2-methylphenyl)phenylmethane;
bis(4-di-p-tolylaminophenyl)phenylmethane;
N,N'-diphenyl-N,N'-di(4-methoxyphenyl)-4,4'-diaminobiphenyl;
N,N,N',N'-tetraphenyl-4,4'-diaminodiphenyl ether; 4,4'-bis(diphenylamino)quaterphenyl;
N,N,N-tri(p-tolyl)amine, 4-(di-p-tolylamino)-4'-[4-(di-p-tolylamino)styryl]stilbene;
4-N,N-diphenylamino-(2-diphenylvinyl)benzene; 3-methoxy-4'-N,N-diphenylaminostylbenzene; N-phenylcarbazole; a compound having two condensed aromatic rings in the molecule described in U.S. Patent No. 5,061,569 such as 4,4'-bis[N-(1-naphthyl)-N-phenylamino]biphenyl (NPD);
and a compound described in Japanese Patent Application Laid-Open Publication No. Hei4-308688, i.e., 4,4',4"-tris[N-(3-methylphenyl)-N-phenylamino]triphenylamine (MTDATA) in which three triphenylamine units are bonded in a starburst

form.

Polymer materials where the above compound(s) are introduced in their polymer chains or are present as their main chains may also be used. Further, an inorganic compound such as p-Si and p-SiC may also be used as the hole-injecting material or the hole-transporting material.

Still further, a hole-transporting material having properties like those of a p-type semiconductor, such as described in Japanese Patent Application Laid-Open Publications Nos. Hei4-297076, 2000-196140 and 2001-102175, J. Appl. Phys., 95, 5773 (2004), Japanese Patent Application Laid-Open Publication No. Hei11-251067, J. Huang et al. (Applied Physics Letters 80 (2002), p. 139) and Japanese Patent Application Laid-Open Publication No. 2003-519432, may also be used.

The hole-transporting layer may be obtained by forming a thin layer using the above hole-transporting material(s) by a known method such as vacuum deposition, spin coating, casting, ink jetting or LB method. The thickness of the hole-transporting layer is not particularly limited, but normally from about 5 nm to 5 μm, and preferably from 5 to 200 nm. The hole-transporting layer may be a single layer composed of one or more kinds of the above materials.

Preferable exemplary compounds of the hole-transporting materials ((1) to (60)) will now be described below, but the present invention is not limited thereto.

[Chemical Formula 1]

(1) 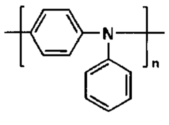

(2) 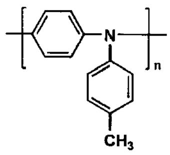

(3) 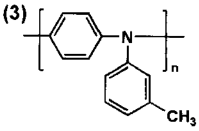

(4) 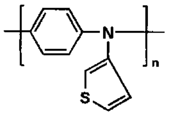

(5) 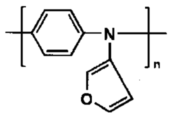

(6) 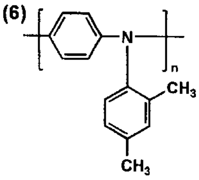

(7) 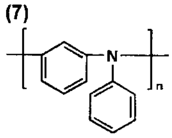

(8) 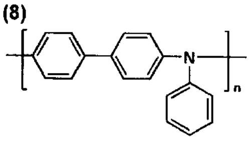

(9) 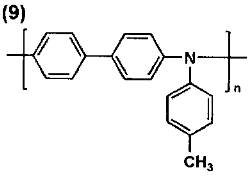

(10) 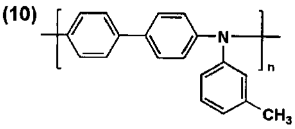

(11) 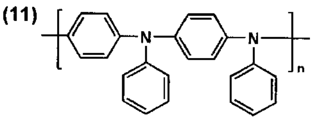

(12) 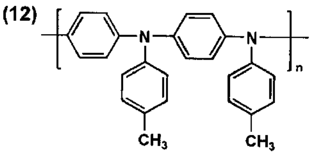

(13) 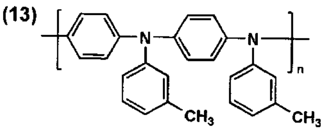

(14) 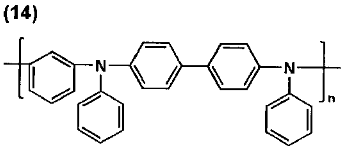

(15) 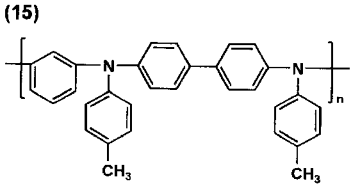

[Chemical Formula 2]

(16)

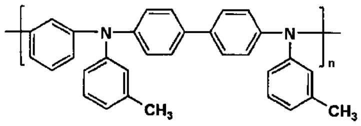

(17)

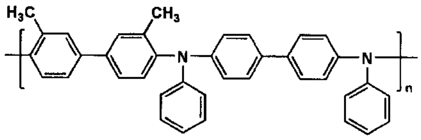

(18)

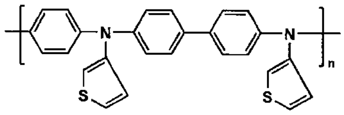

(19)

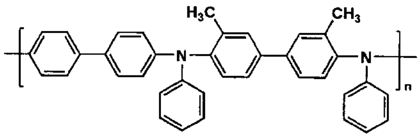

(20)

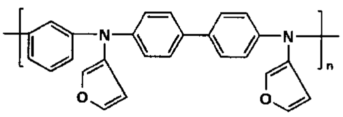

(21)

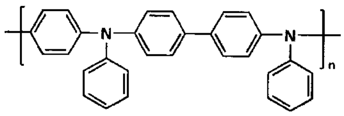

(22)

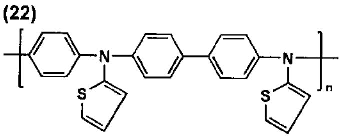

(23)

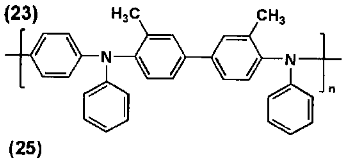

(24)

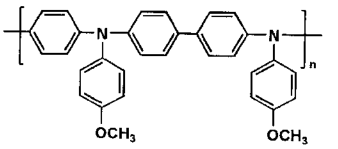

(25)

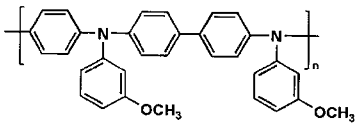

[Chemical Formula 3]

(26)

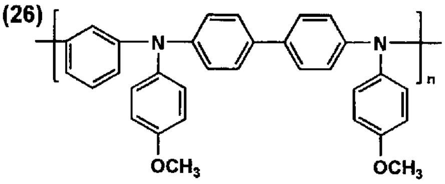

(27)

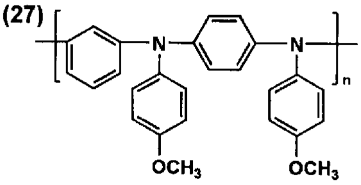

(28)

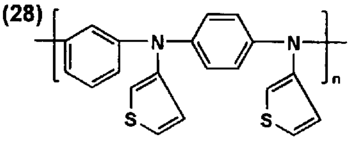

(29)

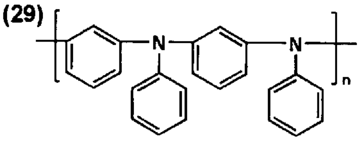

(30)

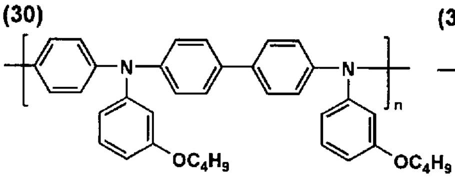

(31)

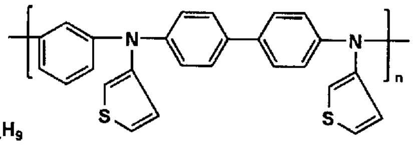

(32)

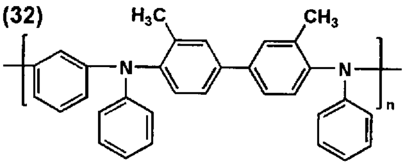

(33)

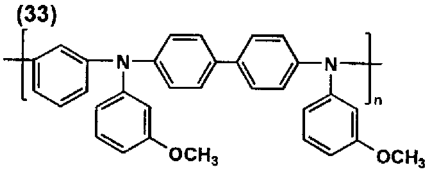

(34)

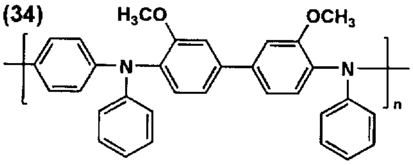

(35)

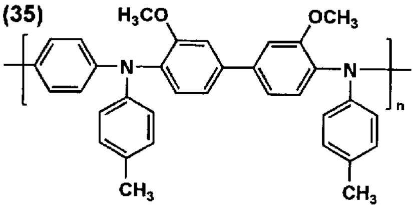

[Chemical Formula 4]

(36)

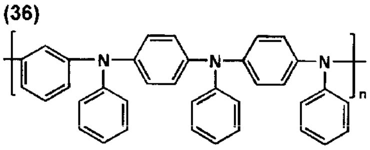

(37)

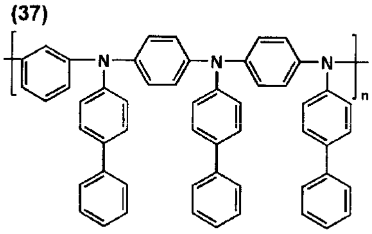

(38)

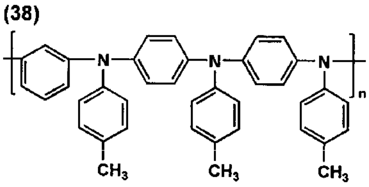

(39)

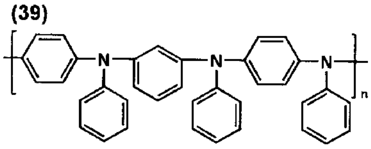

(40)

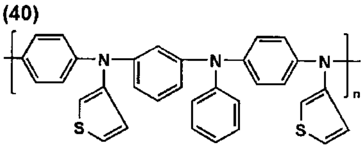

(41)

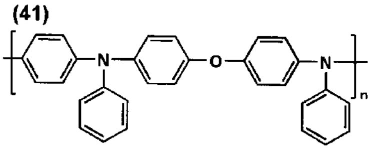

(42)

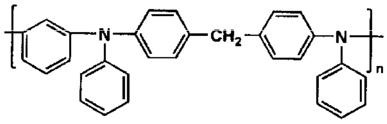

(43)

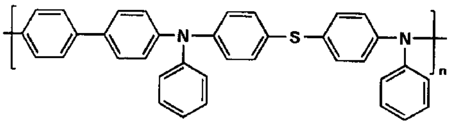

(44)

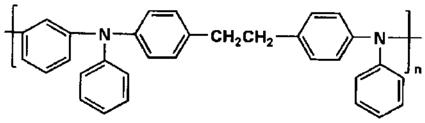

[Chemical Formula 5]

(45)

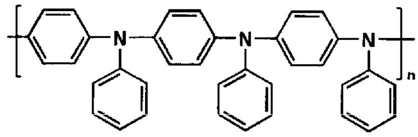

(46)

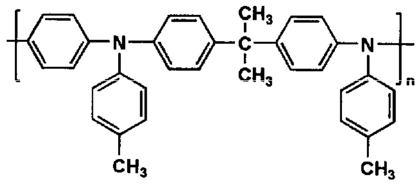

(47)

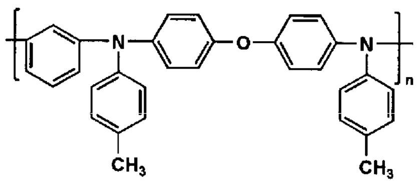

(48)

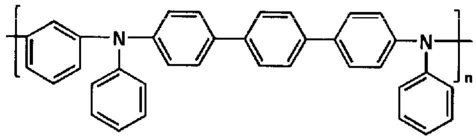

[Chemical Formula 6]

(49)
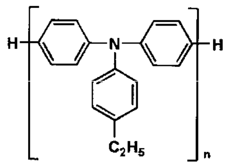
(50)
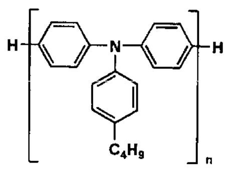
(51)
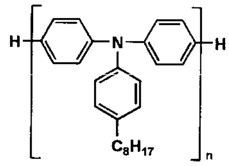

(52)
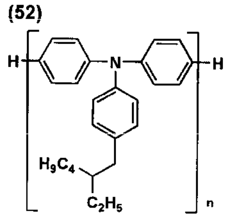
(53)
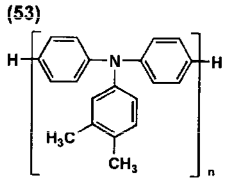
(54)
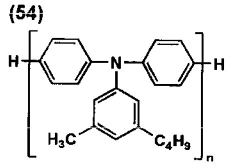

(55)
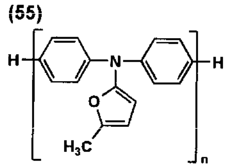
(56)
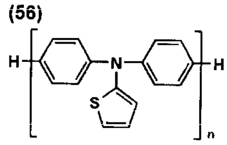
(57)
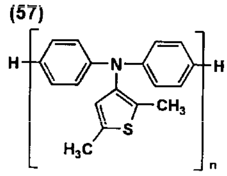

(58)
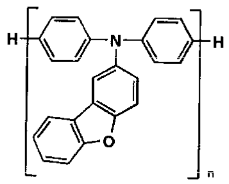
(59)
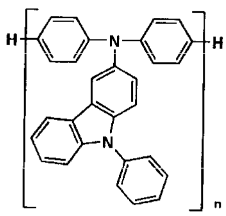

(60)
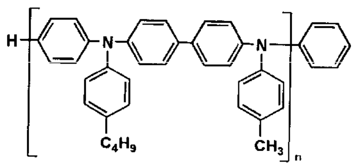

In the above exemplified compounds, n represents a degree of polymerization, and n represents an integer so that a weight-average molecular weight is 50,000 to 200,000. When the weight-average molecular weight is lower than the above range, a material having such a weight-average molecular weight has high solubility in a solvent. There is therefore concern about mixing of such a material into another layer in forming layers, and even if layer formation is completed, efficiency of light emission is low because the molecular weight is low. On the other hand, when the weight-average molecular weight is larger than the above range, this develops problems due to difficulties in synthesis and purification. In such a case, molecular weight distribution shifts to the larger weights and the amount of remaining impurities increases, which leads deterioration in efficiency of light emission, voltage and the length of a life span.

These polymer compounds can be synthesized by a known method described in Makromol. Chem., 193, p.909 (1992), for example.

(3) Electron-Transporting Layer 6

The electron-transporting layer is composed of a material(s) having electron-transporting properties. In a broad sense, an electron-injecting layer and a hole-blocking layer are also classed as the electron-transporting layers. One or more electron-transporting layers may be provided.

Heretofore, in the cases of providing a single or multiple electron-transporting layers, an electron-transporting material(s) (also used as a hole-blocking material) used in the electron-transporting layer that is adjacent to the light-emitting layer on the side of the cathode may be any material(s) having functions for transporting electrons injected from the cathode to the light-emitting layer, and may be selected from conventionally known compounds such as metal complexes, namely, metal complexes of fluorene derivatives, carbazole derivatives, azacarbazole derivatives, oxadiazole derivatives, triazole derivatives, silole derivatives, pyridine derivatives, pyrimidine derivatives, 8-quinolinole derivatives, for example.

In addition, metal-free or metalphthalocyanine, or metal-free or metalphthalocyanine whose end(s) are substituted with an alkyl group(s), sulfonic acid group(s) or the like may be suitably used as the electron-transporting material.

Among them, carbazole derivatives, azacarbazole derivatives, pyridine derivatives are preferable, and azacarbazole derivatives are more preferable for the present invention.

The electron-transporting layer may be obtained by forming a thin layer using the above-described electron-transporting material(s) by a known method such as spin coating, casting, printing such as ink jetting or LB method, and preferably obtained by a wet process using an application liquid (solution) composed of (containing) the above electron-transporting material(s) and fluoroalcohol solvent.

The thickness of the electron-transporting layer is not particularly limited, but normally about 5 nm to 5 μm, and preferably from 5 to 200 nm. The electron-transporting layer may be a single layer composed of one or more kinds of the above material.

Further, an electron-transporting layer doped with impurity(ies) as guest materials in addition to the semiconductor nanoparticles and having high n-type properties may be used. Examples thereof are described in Japanese Patent Application Laid-Open Publications Nos. Hei4-297076, Hei10-270172, 2000-196140 and 2001-102175, and J. Appl. Phys., 95, 5773 (2004), for example.

The electron-transporting layer of the present invention preferably contains an alkali metal salt(s) of an organic compound(s). The organic compound is not particularly limited. Examples include alkali metal salts of formic acid, acetic acid, propionic acid, butyric acid, valeric acid, capronic acid, enanthic acid, caprylic acid, oxalic acid, malonic acid, succinic acid, benzoic acid, phthalic acid, isophthalic acid, telephthalic acid, salicylic acid, pyruvic acid, lactic acid, malic acid, adipic acid, mesylic acid, tosylic acid, benzensulfonic acid; preferably an alkali metal salt of formic acid, acetic acid, propionic acid, butyric acid, valeric acid, capronic acid, enanthic acid, caprylic acid, oxalic acid, malonic acid, succinic acid and benzoic acid; a more preferable example is an alkali metal salt of an aliphatic carboxylic acid, such as formic acid, acetic acid, propionic acid or butyric acid; the number of carbons of the aliphatic carboxylic acid is preferably 4 or less; and most preferable example is an alkali metal salt of acetic acid.

An alkali metal in the alkali metal salt of the organic compound is not particularly limited and is exemplified by Na, K and Cs. K and Cr are preferable, and Cs is more preferable.

The alkali metal salt of an organic compound is exemplified by salts of the above-described organic compounds and the above-described alkali metals, and preferably lithium formate, potassium formate, sodium formate, cesium formate, lithium acetate, potassium acetate, sodium acetate, cesium acetate, lithium propionate, sodium propionate, potassium propionate, cesium propionate, lithium oxalate, sodium oxalate, potassium oxalate, cesium oxalate, lithium malonates, sodium malonate, potassium malonate, cesium malonate, lithium succinate, sodium succinate, potassium succinate, cesium succinate, lithium benzoate, sodium benzoate, potassium benzoate and cesium benzoate, more preferably lithium acetate, potassium acetate, sodium acetate and cesium acetate, and most preferably cesium acetate.

The content of the above doping material(s) is preferably 1.5 to 35% by mass, more preferably 3 to 25% by mass, and most preferably 5 to 15% by mass with respect to the electron-transporting layer to which the doping material(s) are to be added.

(4) Light-Emitting Layer

The light-emitting layer of the organic EL element of the present invention is a layer where electrons and holes injected from the electrodes, the electron-transporting layer and/or the hole-transporting layer are recombined with each other to cause light emission. Light-emitting portions may be within the light-emitting layer or at the interface(s) between the light-emitting layer and its adjacent layer(s).

Configurations of the light-emitting layer of the present invention are not particularly limited as long as the material(s) contained in the light-emitting layer meet the above requirements.

Multiple layers having the same emission spectrum and/or maximum emission wavelength may be provided. It is preferable to provide a non-light-emitting interlayer(s) between the light-emitting layers.

The total thickness as to the light-emitting layer(s) of the present invention is preferably from 15 to 100 nm, and more preferably, to achieve much lower driving voltage, from 15 to 50 nm. In the case of where a non-light-emitting interlayer(s) are provided between the light-emitting layers, the total thickness as to the light-emitting layer(s) in the context of the present invention is the sum of the thicknesses of the light-emitting layers and the non-light-emitting interlayer(s).

The thickness of each light-emitting layer is adjusted to preferably 5 to 50 nm. The relations between the thicknesses of a blue light-emitting layer, a green light-emitting layer and a red light-emitting layer are not particularly limited.

In the present invention, multiple light-emitting materials may be mixed with each other in each light-emitting layer, and a phosphorescence-emitting material(s) and a fluorescence-emitting material(s) may be contained in a single light-emitting layer.

Preferably, the light-emitting layer of the present invention contains a host compound(s) and a light-emitting material(s) (also referred to as a light-emitting dopant compound(s)), and light is emitted from the light-emitting material(s).

(4.1) Host Compound

The host compound contained in the light-emitting layer of the organic EL element of the present invention is a compound having a phosphorescence quantum yield in phosphorescence emission at room temperature (25°C) of preferably less than 0.1, and more preferably less than 0.01. The volume ratio of the host compound(s) is preferably 50% or more with respect to all of the compound(s) contained in the light-emitting layer.
One known host compound may be used alone or two or more kinds of known compounds may be used together. By using two or more kinds of host compounds, charge transfer can be controlled, and thus a more efficient organic EL element is provided. When multiple kinds of light-emitting materials described later are used, light of different colors can be mixed, and thus any desired light colors can be obtained.
The host compound used in the present invention may be a low-molecular-weight compound, a polymer containing a repeating unit(s), and a low-molecular-weight compound containing a polymerizable group(s) such as a vinyl group and epoxy group (i.e., a polymerizable light-emitting host). When a polymer material(s) are used, their purification is difficult and swelling, gelation or the like, which is thought to be difficult to release a solvent, is caused. To prevent them, the molecular weight is preferably not large. Specifically, it is preferable to use a material that has a molecular weight of, during its application, 2000 or less, more preferably 1000 or less, and further more preferably 800 or less.
Preferably, the known host compound has hole-transporting properties and electron-transporting properties, prevents light wavelength from being lengthened and has high Tg (glass transition temperature). Grass transition temperature (Tg) is obtained by a method according to JIS-K-7121 by DSC (Differential Scanning Colorimetry).
Specific examples of the known host compounds include compounds described in, for example, Japanese Patent Application Laid-open Publications Nos.2001-257076, 2002-308855, 2001-313179, 2002-319491, 2001-357977, 2002-334786, 2002-8860, 2002-334787, 2002-15871, 2002-334788, 2002-43056, 2002-334789, 2002-75645, 2002-338579, 2002-105445, 2002-343568, 2002-141173, 2002-352957, 2002-203683, 2002-363227, 2002-231453, 2003-3165, 2002-234888, 2003-27048, 2002-255934, 2002-260861, 2002-280183, 2002-299060, 2002-302516, 2002-305083, 2002-305084 and 2002-308837.
The host compound used in the present invention is preferably a carbazole derivative.
Preferably, a compound represented by the general formula (1) is used as the host compound.
[Chemical Formula 7]

General formula (1)

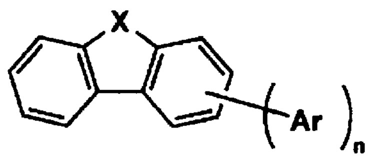

In the general formula (1), "X" represents NR’, O, S, CR’R" or SiR’R";
"R"’ and "R"" each represent a hydrogen atom or a substituent;
"Ar" represents an aromatic ring; and
"n" represents an integer from 0 to 8.
In X of the general formula (1), examples of substituents each represented by R’ or R" include alkyl groups (such as methyl group, ethyl group, propyl group, isopropyl group, t-butyl group, pentyl group, hexyl group, octyl group, dodecyl group, tridecyl group, tetradecyl group and pentadecyl group), cycloalkyl groups (such as cyclopentyl group and cyclohexyl group), alkenyl groups (such as vinyl group and allyl group), alkynyl groups (such as ethynyl group and propargyl group), aromatic hydrocarbon ring groups (also referred to as aromatic carbon ring groups or aryl groups, such as phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group and biphenyl group), aromatic hetero ring groups (such as pyridyl group, pyrimidinyl group, furyl group, pyrrolyl group, imidazolyl group, benzoimidazolyl group, pyrazolyl group, pyrazinyl group, triazolyl group (exemplified by 1,2,4-triazole-1-yl group and 1,2,3-triazole-1-yl group), oxazolyl group, benzoxazolyl group, thiazolyl group, isoxazolyl group, isothiazolyl group, furazanyl group, thienyl group, quinolyl group, benzofuryl group, dibenzofuryl group, benzothienyl group, dibenzothienyl group, indolyl group, carbazolyl group, carbolinyl group, diazacarbazolyl group (a group where one of the carbon atoms of the carboline ring of the carbolinyl group is substituted with a nitrogen atom), quinoxalinyl group, pyridazinyl group, triazinyl group, quinazolinyl group and phthalazinyl group), hetero ring groups (such as pyrrolidyl group, imidazolidyl

group, morpholyl group and oxazolidyl group), alkoxy groups (such as methoxy group, ethoxy group, propyloxy group, pentyloxy group, hexyloxy group, octyloxy group and dodecyloxy group), cycloalkoxy groups (such as cyclopentyloxy group and cyclohexyloxy group), aryloxy groups (such as phenoxy group and naphthyloxy group), alkylthio groups (such as methylthio group, ethylthio group, propylthio group, pentylthio group, hexylthio group, octylthio group and dodedcylthio group), cycloalkylthio groups (such as cyclopentylthio group and cyclohexylthio group), arylthio groups (such as phenylthio group and naphthylthio group), alkoxycarbonyl groups (such as methyloxycarbonyl group, ethyloxycarbonyl group, butyloxycarbonyl group, octyloxycarbonyl group and dodecyloxycarbonyl group), aryloxycarbonyl groups (such as phenyloxycarbonyl group and naphthyloxycarbonyl group), sulfamoyl groups (such as aminosulfonyl group, methylaminosulfonyl group, dimethylaminosulfonyl group, butylaminosulfonyl group, hexylaminosulfonyl group, cyclohexylaminosulfonyl group, octylaminosulfonyl group, dodecylaminosulfonyl group, phenylaminosulfonyl group, naphthylaminosulfonyl group and 2-pyridylaminosulfonyl group), acyl groups (such as acetyl group, ethylcarbonyl group, propylcarbonyl group, pentylcarbonyl group, cyclohexylcarbonyl group, octylcarbonyl group, 2-ethylhexylcarbonyl group, dodecylcarbonyl group, phenylcarbonyl group, naphthylcarbonyl group and pyridylcarbonyl group), acyloxy groups (such as acetyloxy group, ethylcarbonyloxy group, butylcarbonyloxy group, octylcarbonyloxy group, dodecylcarbonyloxy group and phenylcarbonyloxy group), amide groups (such as methylcarbonylamino group, ethylcarbonylamino group, dimethylcarbonylamino group, propylcarbonylamino group, pentylcarbonylamino group, cyclohexylcarbonylamino group, 2-ethyhexylcarbonylamino group, octylcarbonylamino group, dodecylcarnobylamino group, phenylcarbonylamino group and naphthylcarbonylamino group), carbamoyl groups (such as aminocarbonyl group, methylaminocarbonyl group, dimethylaminocarbonyl group, propylaminocarbonyl group, pentylaminocarbonyl group, cyclohexylcarbonylamino group, octylaminocarbonyl group, 2-ethylhexylaminocarbonyl group, dodecylaminocarbonyl group, phenylaminocarbonyl group, naphthylaminocarbonyl group and 2-pyridylaminocarbonyl group), ureido groups (such as methylureido group, ethylureido group, pentylureido group, cyclohexylureido group, octylureido group, dodecylureido group, phenylureido group, naphthylureido group and 2-pyridylaminoureido group), sulfinyl groups (such as methylsulfinyl group, ethylsulfinyl group, butylsulfinyl group, cyclohexylsulfinyl group, 2-ethylhexylsulfinyl group, dodecylsulfinyl group, phenylsulfinyl group, naphtylsulfinyl group and 2-pyridylsulfinyl group), alkylsulfonyl groups (such as methylsulfonyl group, ethylsulfonyl group, butylsulfonyl group, cyclohexylsulfonyl group, 2-ethylhexylsulfonyl group and dodecylsulfonyl group), arylsulfonyl or heteroarylsulfonyl groups (such as phenylsulfonyl group, naphthylsulfonyl group and 2-pyridylsulfonyl group), amino groups (such as amino group, ethylamino group, dimethylamino group, butylamino group, cyclopentylamino group, 2-ethylhexylamino group, dodecylamino group, anilino group, naphthylamino group and 2-pyridylamino group), halogen atoms (such as fluorine atom, chlorine atom and bromine atom), fluorohydrocarbon groups (such as fluoromethyl group, trifluoromethyl group, pentafluoroethyl group and pentafluorophenyl group), cyano group, nitro group, hydroxy group, mercapto group, silyl groups (such as trimethylsilyl group, triisopropylsilyl group, triphenylsilyl group and phenyldiethylsilyl group). These substituents may be substituted with the above substituent(s). These substituents may be bonded to each other to form a ring(s).

Among them, X is preferably NR' or O. Particularly preferable examples of R' are aromatic hydrocarbon ring groups (also referred to as aromatic carbon ring groups or aryl groups, such as phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group and biphenyl group) and aromatic hetero ring groups (such as furyl group, thienyl group, pyridyl group, pyridazinyl group, pyrimidinyl group, pyrazinyl group, triazinyl group, imidazolyl group, pyrazolyl group, thiazolyl group, quinazolinyl group and phthalazinyl group).

These aromatic hydrocarbon groups and aromatic hetero ring groups may be substituted with a substituent(s) represented by R' or R" in X of the general formula (1).

Examples of the aromatic ring represented by Ar in the general formula (1) include aromatic hydrocarbon rings and aromatic hetero rings. Such an aromatic ring may be monocyclic or a fused ring, and may be non-substituted or substituted with a substituent(s) each represented by R' or R" in X of the general formula (1).

In the general formula (1), examples of the aromatic hydrocarbon ring groups represented by Ar include benzene ring, biphenyl ring, naphthalene ring, azulene ring, anthracene ring, phenanthrene ring, pyrene ring, chrysene ring, naphthacene ring, triphenylene ring, o-terphenyl ring, *m*-terphenyl ring, *p*-terphenyl ring, acenaphthene ring, coronene ring, fluorene ring, fluoranthrene ring, naphthacene ring, pentacene ring, perylene ring, pentaphene ring, picene ring, pyrene ring, pyranthrene ring, anthranthrene ring. These rings may be substituted with a substituent(s) each represented by R' or R" in X of the general formula (a).

In the partial structure represented by the general formula (1), examples of the aromatic hetero ring groups represented by Ar include furan ring, dibenzofuran ring, thiophene ring, oxazole ring, pyrrole ring, pyridine ring, pyridazine ring, pyrimidine ring, pyrazine ring, triazine ring, benzoimidazole ring, oxadiazole ring, triazole ring, imidazole ring, pyrazole ring, thiazole ring, indole ring, indazole ring, benzoimidazole ring, benzothiazole ring, benzoxazole ring, quinoxaline ring, quinazoline ring, cinnoline ring, quinoline ring, isoquinoline ring, phthalazine ring, naphthyridine ring, carbazole ring, carboline ring and diazacarbazole ring (a ring where one carbon atom of the hydrocarbon ring constituting the carboline ring is substituted with a nitrogen atom).

These rings may be substituted with a substituent(s) each represented by R' or R" in X of the general formula (1).

Among them, preferable the aromatic ring represented by Ar of the general formula (1) are carbazole ring, carboline ring, dibenzofuran ring and benzene ring. A substituted benzene ring is more preferable, and a benzene ring containing a carbazolyl group(s) is particularly preferable.

A preferable example of the aromatic ring represented by Ar of the general formula (1) is a fused ring derived from 3 or more rings. Examples of the fused aromatic hydrocarbon ring where 3 or more rings are fused together include naphthacene ring, anthracene ring, tetracene ring, pentacene ring, hexacene ring, phenanthrene ring, pyrene ring, benzopyrene ring, benzoazulene ring, chrysene ring, benzochrysene ring, acenaphthene ring, acenaphthylene ring, triphenylene ring, coronene ring, benzocoronene ring, hexabenzocoronene ring, fluorene ring, benzofluorene ring, fluoranthene ring, perylene ring, naphthoperylene ring, pentabenzoperylene ring, benzoperylene ring, pentaphene ring, picene ring, pyranthrene ring, coronene ring, naphthocoronene ring, ovalene ring anthranthrene ring. These rings may be substituted with the above substituent(s).

Examples of the aromatic hetero ring where 3 or more rings are fused together include acridine ring, benzoquinoline ring, carbazole ring, carboline ring, phenazine ring, phenanthridine ring, phenanthroline ring, carboline ring, cyclazine ring, quindoline ring, tepenijine ring, quinindoline ring, triphenodithiazine ring, triphenodioxazine ring, phenanthridine, ring, anthrazine ring, perimidine ring, diazacarbazole ring (a ring where one carbon atom of the hydrocarbon ring constituting the carboline ring is substituted with a nitrogen atom), phenanthroline ring, dibenzofuran ring, dibenzothiophene ring, naphthofuran ring, naphthothiophene ring, benzodifuran ring, benzothiophene ring, naphthodifuran ring, naphthodithiophene ring, anthrafuran ring, anthradifuran ring, anthrathiophene ring, anthradithiophene ring, thianthrene ring, phenoxathiin ring and thiophanthrene ring (naphthothiophene ring). These rings may be substituted with a substituent(s).

In the general formula (1), n represents an integer from 0 to 8, and preferably from 0 to 2. In the case where X is O or S, n is preferably 1 or 2.

In the present invention, the host compound containing a dibenzofuran ring(s) and a carbazole ring(s) is particularly preferable.

Specific exemplary compounds of the host compounds represented by the general formula (1) (a-1 to a-41) are described below, but the present invention is not limited thereto.

[Chemical Formula 8]

a-1

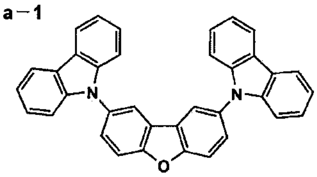

a-2

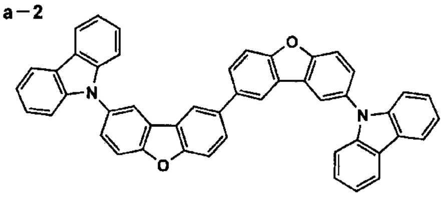

a-3

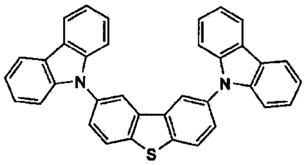

a-4

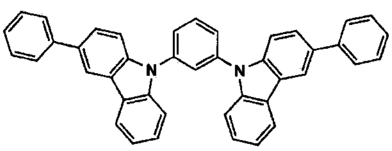

a-5

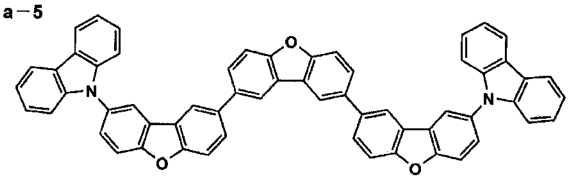

a-6

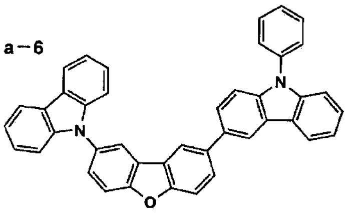

[Chemical Formula 9]

a-7

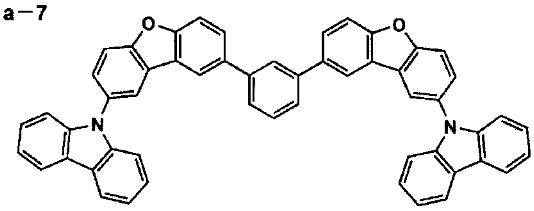

a-8

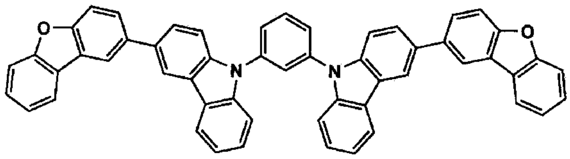

a-9

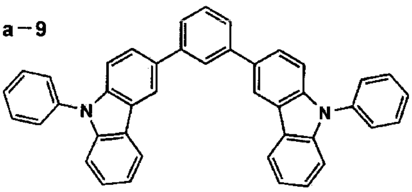

a-10

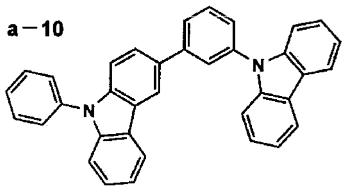

a-11

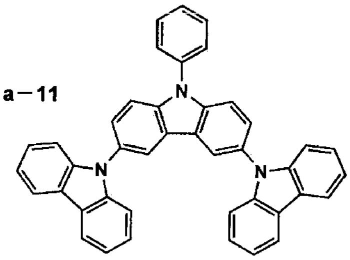

a-12

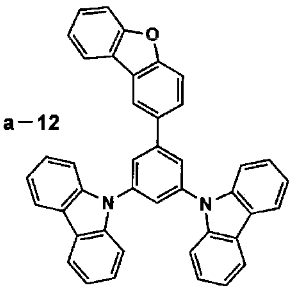

[Chemical Formula 10]

a－13

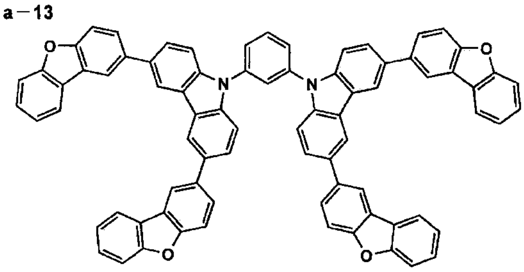

a－14

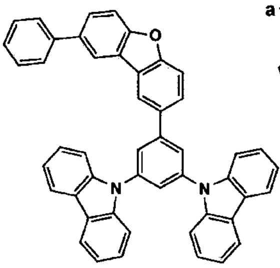

a－15

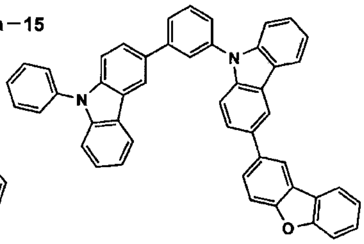

a－16

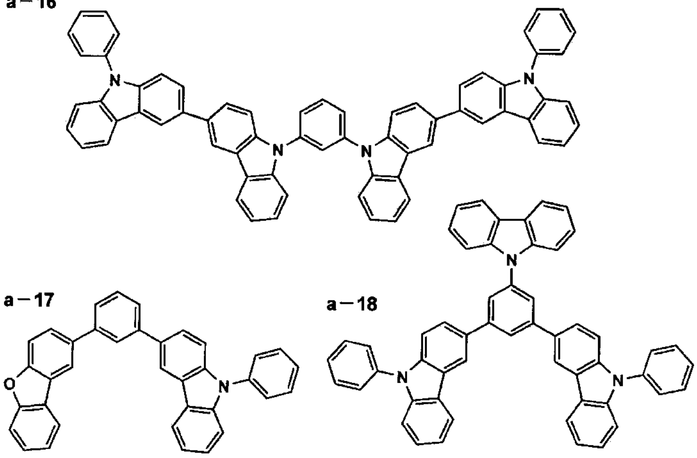

a－17

a－18

[Chemical Formula 11]

a-19

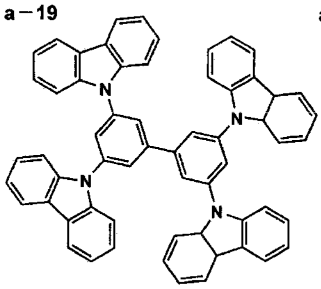

a-20

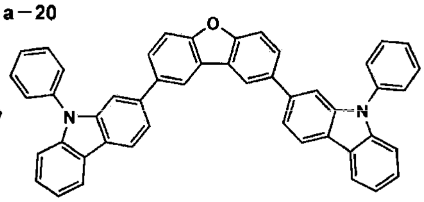

a-21

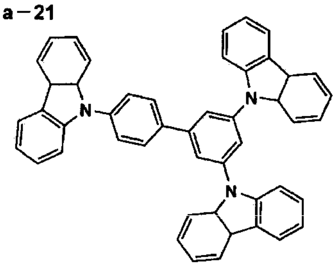

a-22

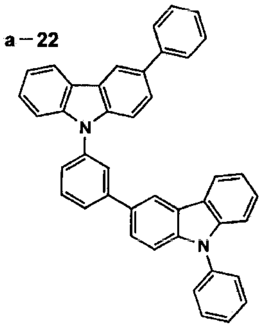

a-23

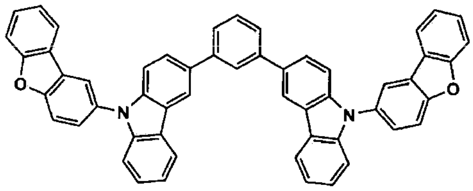

a-24

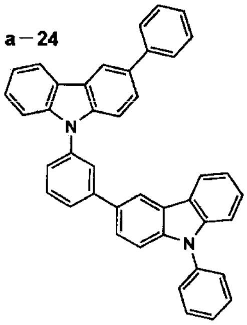

[Chemical Formula 12]

**a－25**

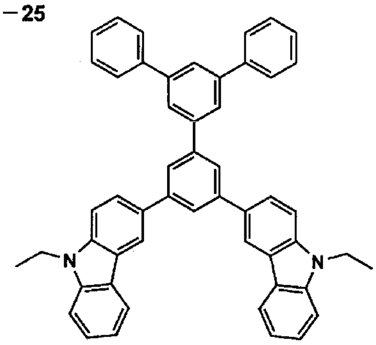

**a－26**

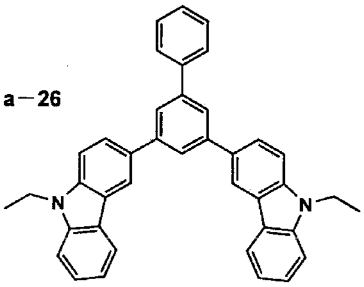

**a－27**

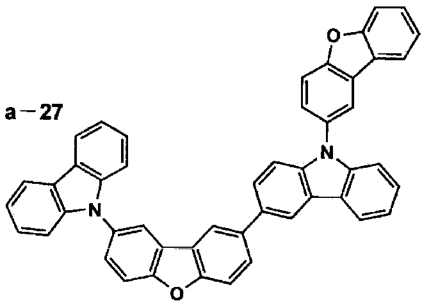

**a－28**

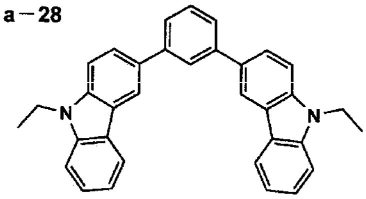

**a－29**

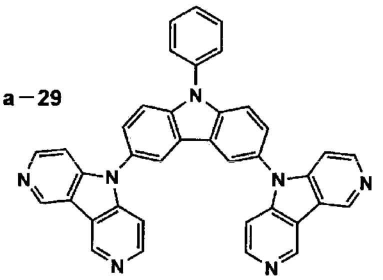

[Chemical Formula 13]

a−30

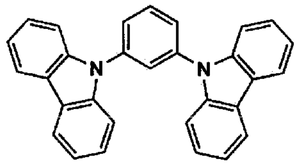

a−31

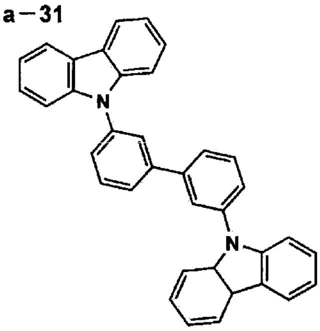

a−32

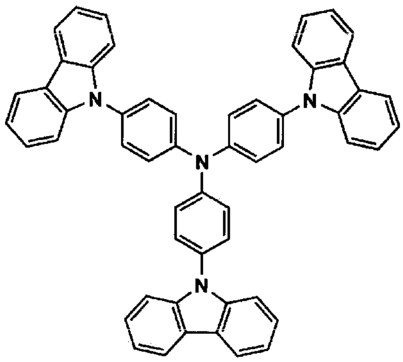

a−33

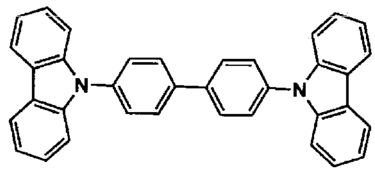

a−34

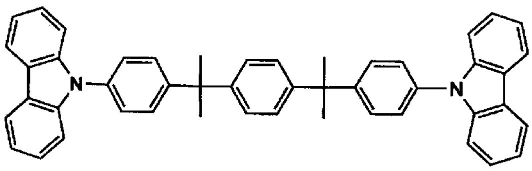

[Chemical Formula 14]

a−35

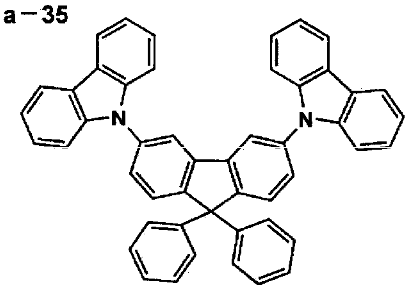

a−36

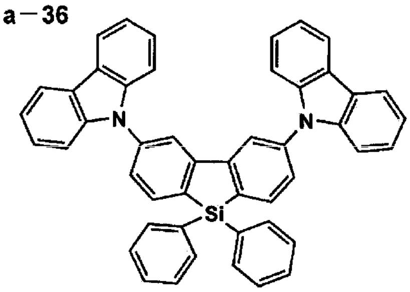

[Chemical Formula 15]

a-37

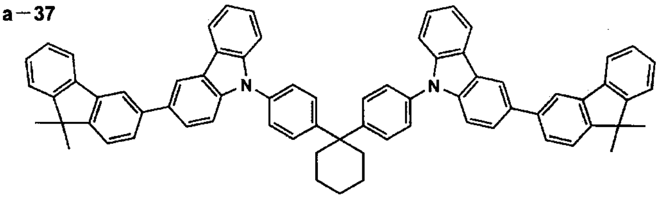

a-38

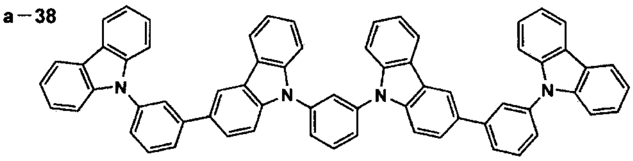

a-39

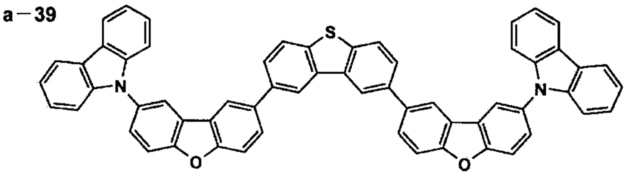

a-40

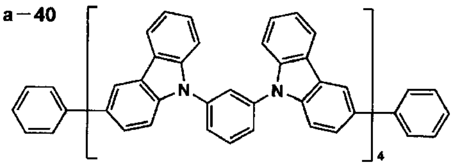

a-41

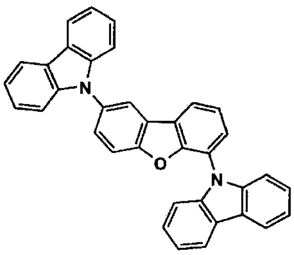

(4.2) Light-Emitting Material (Light-Emitting Dopant)

As a light-emitting material (light-emitting dopant) of the present invention, a fluorescent compound(s) and a phosphorescent material(s) (also referred to as a phosphorescent compound(s) or a phosphorescence-emitting compound(s)) may be used, and a phosphorescent material is preferable.

In the present invention, a phosphorescent material is a compound showing light emission from an excited triplet state. Specifically, the phosphorescent material is a compound which emits phosphorescence at room temperature (25°C) and is defined as having a phosphorescence quantum yield at 25°C of 0.01 or more, and preferably 0.1 or more.

A phosphorescence quantum yield may be measured according to the method described in page 398 of the fourth series of Experimental Chemistry 7, Spectroscopy II, 1992 from MARUZEN Co., Ltd. Various solvents may be used in measuring a phosphorescence quantum yield in a solution. The phosphorescent material of the present invention may be any compound as long as a phosphorescence quantum yield of the phosphorescent dopant satisfies the above requirement (0.01 or more).

There are two principles of light emission by a phosphorescent material. One is an energy transfer-type, wherein the recombination of carriers occurs on a host compound onto which the carriers are transferred to produce an excited state of the host compound, and then via transfer of this energy to a phosphorescent material, light emission from the phosphorescent material occurs. The other is a carrier trap-type, wherein a phosphorescent material serves as a carrier trap to cause recombination of carriers on the phosphorescent material, and thereby light emission from the phosphorescent material occurs. In each type, the energy in the excited state of the phosphorescent material is required to be lower than that in the excited state of the host compound.

The phosphorescent material may be a compound selected from known compounds that have been used in a light-emitting layer of an organic EL element as needed, and is preferably a complex compound containing a metal of Groups 8 to 10 of the periodic table, more preferably an iridium compound, an osmium compound, a platinum compound (platinum complex compound) or a rare earth complex, and most preferably an iridium compound.

In the embodiments, the phosphorescent materials include at least one blue phosphorescent material, and preferably include at least one blue phosphorescent material and a phosphorescent material(s) having a band gap lower than that of the blue phosphorescent material.

The phosphorescent dopant of the present invention which dopant is represented by the general formula (2) will now be described in detail.

[Chemical Formula 16]

General formula (2)

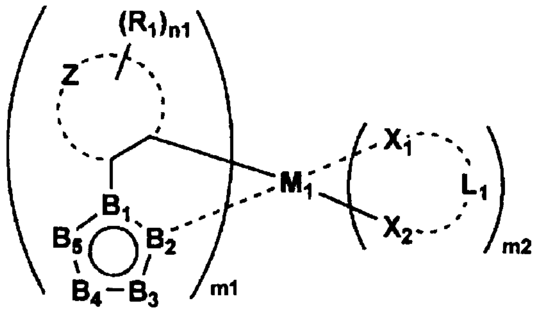

In the general formula (2), "$R_1$" represents a substituent;
"Z" represents a group of non-metal atoms necessary for forming a five to seven-membered ring;
"n1" represents an integer from 0 to 5;
"B1 to B5" each represent a carbon atom, a nitrogen atom, an oxygen atom or a sulfur atom and at least one of B1 to B5 represents a nitrogen atom;
"$M_1$" represents a transition metal element of Groups 8 to 10 of the periodic table;
"$X_1$" and "$X_2$" each represent a carbon atom, a nitrogen atom or an oxygen atom and "$L_1$" represents a group of atoms forming a bidentate ligand together with $X_1$ and $X_2$; and
"m1" represents an integer 1, 2 or 3, "m2" represents an integer 0, 1 or 2 and "m1 + m2" is equal to 2 or 3.

The phosphorescent compound of the present invention represented by the general formula (2) has a HOMO of -5.15 to -3.50 eV and a LUMO of -1.25 to +1.00 EV, and preferably has a HOMO of -4.80 to -3.50 eV and a LUMO of -0.80 to +1.00 eV.

In the phosphorescent compound represented by the general formula (2), examples of substituents represented by $R_1$ include alkyl groups (such as methyl group, ethyl group, propyl group, isopropyl group, *tert*-butyl group, pentyl group, hexyl group, octyl group, dodecyl group, tridecyl group, tetradecyl group and pentadecyl group), cycloalkyl groups (such as cyclopentyl group and cyclohexyl group), alkenyl groups (such as vinyl group and allyl group), alkynyl groups (such as ethynyl group and propargyl group), aromatic hydrocarbon ring groups (also referred to as aromatic carbon ring groups or aryl groups, such as phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group and biphenyl group), aromatic hetero ring groups (such as pyridyl group, pyrimidinyl group, furyl group, pyrrolyl group, imidazolyl group, benzoimidazolyl group, pyrazolyl group, pyrazinyl group, triazolyl group (exemplified by 1,2,4-triazole-1-yl group and 1,2,3-triazole-1-yl group), oxazolyl group, benzoxazolyl group, thiazolyl group, isoxazolyl group, isothiazolyl group, furazanyl group, thienyl group, quinolyl group, benzofuryl group, dibenzofuryl group, benzothienyl group, dibenzothienyl group, indolyl group, carbazolyl group, carbolinyl group, diazacarbazolyl group (a group where one of the carbon atoms of the carboline ring of the carbolinyl group is substituted with a nitrogen atom), quinoxalinyl group, pyridazinyl group, triazinyl group, quinazolinyl group and phthalazinyl group), hetero ring groups (such as pyrrolidyl group, imidazolidyl group, morpholyl group and oxazolidyl group), alkoxy groups (such as methoxy group, ethoxy group, propyloxy group, pentyloxy group, hexyloxy group, octyloxy group and dodecyloxy group), cycloalkoxy groups (such as cyclopentyloxy group and cyclohexyloxy group), aryloxy groups (such as phenoxy group and naphthyloxy group), alkylthio groups (such as methylthio group, ethylthio group, propylthio group, pentylthio group, hexylthio group, octylthio group and dodedcylthio group), cycloalkylthio groups (such as cyclopentylthio group and cyclohexylthio group), arylthio groups (such as phenylthio group and naphthylthio group), alkoxycarbonyl groups (such as methyloxycarbonyl group, ethyloxycarbonyl group, butyloxycarbonyl group, octyloxycarbonyl group and dodecyloxycarbonyl group), aryloxycarbonyl groups (such as phenyloxycarbonyl group and naphthyloxycarbonyl group), sulfamoyl groups (such as aminosulfonyl group, methylaminosulfonyl group, dimethylaminosulfonyl group, butylaminosulfonyl group, hexylaminosulfonyl group, cyclohexylaminosulfonyl group, octylaminosulfonyl group, dodecylaminosulfonyl group, phenylaminosulfonyl group, naphthylaminosulfonyl group and 2-pyridylaminosulfonyl group), acyl groups (such as acetyl group, ethylcarbonyl group, propylcarbonyl group, pentylcarbonyl group, cyclohexylcarbonyl group, octylcarbonyl group, 2-ethylhexylcarbonyl group,

dodecylcarbonyl group, phenylcarbonyl group, naphthylcarbonyl group and pyridylcarbonyl group), acyloxy groups (such as acetyloxy group, ethylcarbonyloxy group, butylcarbonyloxy group, octylcarbonyloxy group, dodecylcarbonyloxy group and phenylcarbonyloxy group), amide groups (such as methylcarbonylamino group, ethylcarbonylamino group, dimethylcarbonylamino group, propylcarbonylamino group, pentylcarbonylamino group, cyclohexylcarbonylamino group, 2-ethyhexylcarbonylamino group, octylcarbonylamino group, dodecylcarnobylamino group, phenylcarbonylamino group and naphthylcarbonylamino group), carbamoyl groups (such as aminocarbonyl group, methylaminocarbonyl group, dimethylaminocarbonyl group, propylaminocarbonyl group, pentylaminocarbonyl group, cyclohexylcarbonylamino group, octylaminocarbonyl group, 2-ethylhexylaminocarbonyl group, dodecylaminocarbonyl group, phenylaminocarbonyl group, naphthylaminocarbonyl group and 2-pyridylaminocarbonyl group), ureido groups (such as methylureido group, ethylureido group, pentylureido group, cyclohexylureido group, octylureido group, dodecylureido group, phenylureido group, naphthylureido group and 2-pyridylaminoureido group), sulfinyl groups (such as methylsulfinyl group, ethylsulfinyl group, butylsulfinyl group, cyclohexylsulfinyl group, 2-ethylhexylsulfinyl group, dodecylsulfinyl group, phenylsulfinyl group, naphtylsulfinyl group and 2-pyridylsulfinyl group), alkylsulfonyl groups (such as methylsulfonyl group, ethylsulfonyl group, butylsulfonyl group, cyclohexylsulfonyl group, 2-ethylhexylsulfonyl group and dodecylsulfonyl group), arylsulfonyl or heteroarylsulfonyl groups (such as phenylsulfonyl group, naphthylsulfonyl group and 2-pyridylsulfonyl group), amino groups (such as amino group, ethylamino group, dimethylamino group, butylamino group, cyclopentylamino group, 2-ethylhexylamino group, dodecylamino group, anilino group, naphthylamino group and 2-pyridylamino group), cyano group, nitro group, hydroxy group, mercapto group, silyl groups (such as trimethylsilyl group, triisopropylsilyl group, triphenylsilyl group and phenyldiethylsilyl group). Among these substituents, alkyl group and aryl group are preferable.

Z represents a group of non-metal atoms necessary for forming a five to seven-membered ring. Example of the five to seven-membered ring formed with Z include benzene ring, naphthalene ring, pyridine ring, pyrimidine ring, pyrrole ring, thiophene ring, pyrazole ring, imidazole ring, oxazole ring and thiazole ring. Among them, benzene ring is preferable.

$B_1$ to $B_5$ each represent a carbon atom, a nitrogen atom, an oxygen atom or a sulfur atom and at least one of $B_1$ to $B_5$ represents a nitrogen atom. An aromatic hetero ring formed with $B_1$ to $B_5$ is preferably monocyclic. Examples of the aromatic hetero ring include pyrrole ring, pyrazole ring, imidazole ring, triazole ring, tetrazole ring, oxazole ring, isoxazole ring, thiazole ring, isothiazole ring, oxadiazole ring and thiadiazole ring. Among them, pyrazole ring and imidazole ring are preferable. Imidazole ring where $B_2$ and $B_5$ are nitrogen atoms is particularly preferable. The above-described rings may be substituted with the above substituent(s). Such a substituent is preferably alkyl group or aryl group, and more preferably aryl group.

$L_1$ represents a group of atoms forming a bidentate ligand together with $X_1$ and $X_2$. Examples of the bidentate ligand represented by $X_1$-$L_1$-$X_2$ include phenylpyridine, phenylpyrazole, phenylimidazole, phenyltriazole, phenyltetrazole, pyrazabole, picolinic acid and acetyl acetone. These compounds may be substituted or non-substituted.

In the formula, m1 represents an integer from 1 to 3; m2 represents an integer from 0 to 2; and m1 + m2 equals to 2 or 3. Preferably, m2 is 0. As a metal represented by M1, a transition metal element of Groups 8 to 10 of the periodic table (also referred to simply as a transition metal) is used, and iridium and platinum are preferable. Iridium is more preferable.

When a nitrogen-containing hetero ring formed with $B_1$ to $B_5$ is an imidazole ring, the general formula (2) is preferably represented by the general formula (3).

[Chemical Formula 17]

General formula (3)

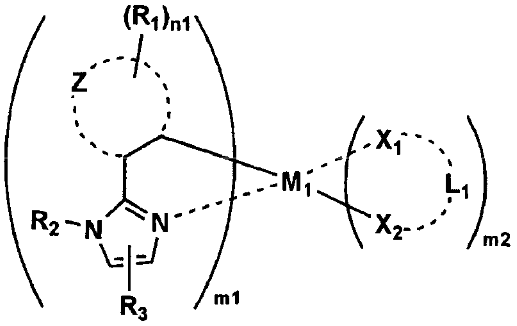

In the general formula (3), "$R_1$", "$R_2$" and "$R_3$" each represent a substituent;
"Z" represents a group of non-metal atoms necessary for forming a five to seven-membered ring;
"n1" represents an integer from 0 to 5;

"$M_1$" represents a transition metal element of Groups 8 to 10 of the periodic table;
"$X_1$" and "$X_2$" each represent a carbon atom, a nitrogen atom or an oxygen atom and "$L_1$" represents a group of atoms forming a bidentate ligand together with $X_1$ and $X_2$; and
"m1" represents an integer 1, 2 or 3, "m2" represents an integer 0, 1 or 2 and "m1 + m2" is equal to 2 or 3.

In the general formula (3), substituents represented by "$R_1$", "$R_2$" and "$R_3$" correspond with the substituent represented by $R_1$ of the general formula (2).

In the general formula (3), Z, $M_1$, $X_1$, $X_2$ and $L_1$ correspond with those of the general formula (2), and also m1 and m2 correspond with those of the general formula (2).

The substituent represented by $R_2$ of the general formula (3) is preferably an aromatic hydrocarbon ring group (aromatic carbon ring group). A substituted aryl group is preferable, and a group represented by the general formula (4) is preferable as the substituted aryl group.

[Chemical Formula 18]

General formula (4)

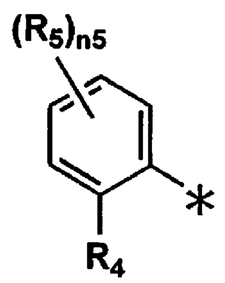

In the general formula (4), "$R_4$" represents a substituent with a steric parameter (Es) of -0.5 or less, "$R_5$" represents an integer from 0 to 5, and "*" represents a linking position.

Specific exemplary compounds of the phosphorescent compounds represented by the general formula (2) (D-1 to D-133) will now be described, but the present invention is not limited thereto.

[Chemical Formula 19]

D-1
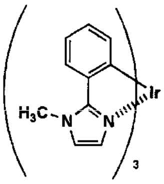
D-2
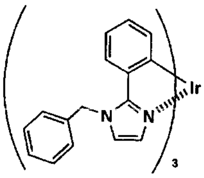
D-3
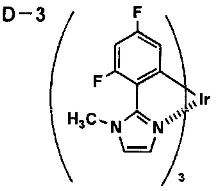
D-4
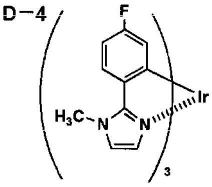
D-5
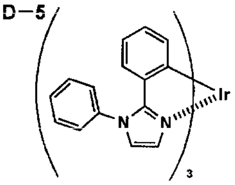
D-6
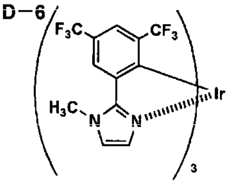
D-7
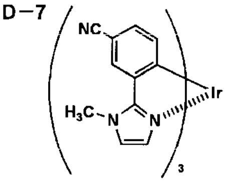
D-8
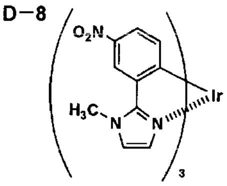
D-9
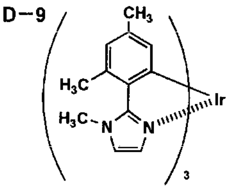
D-10
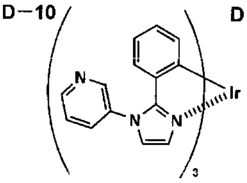
D-11
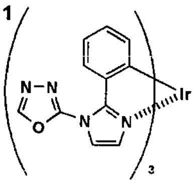
D-12
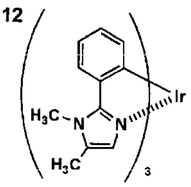
D-13
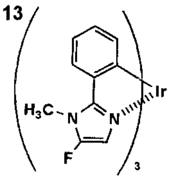
D-14
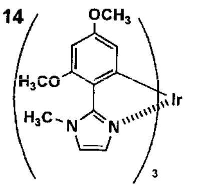
D-15
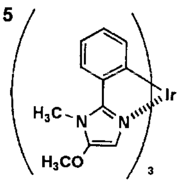

[Chemical Formula 20]

D-16
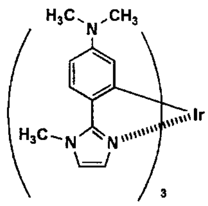

D-17
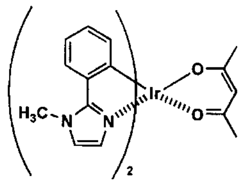

D-18
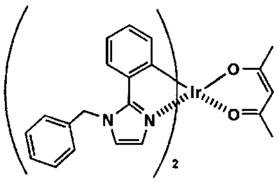

D-19
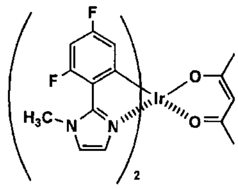

D-20
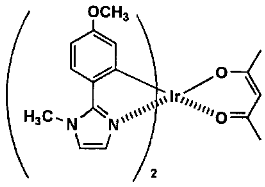

D-21
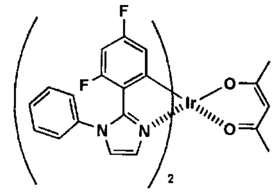

D-22
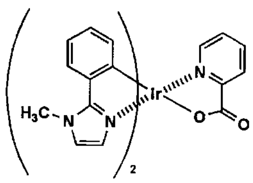

D-23
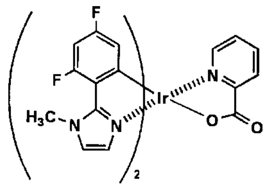

D-24
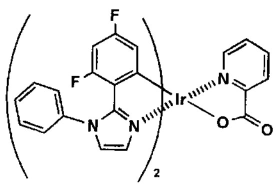

D-25
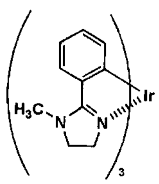

[Chemical Formula 21]

D-26

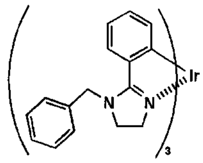

D-27

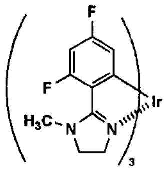

D-28

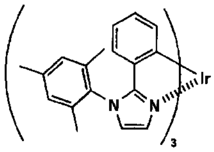

D-29

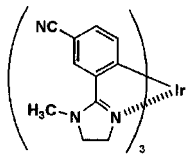

D-30

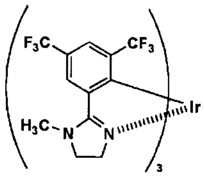

D-31

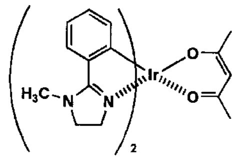

D-32

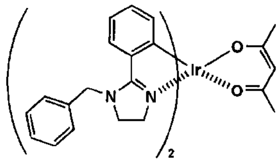

D-33

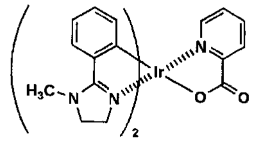

D-34

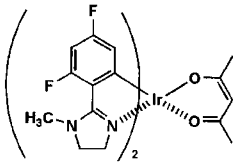

D-35

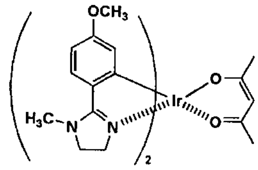

D-36

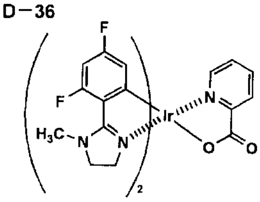

D-37

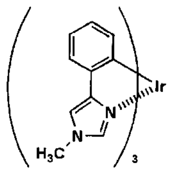

D-38

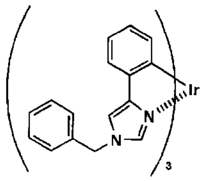

[Chemical Formula 22]

D-39
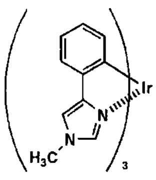
D-40
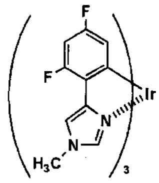
D-41
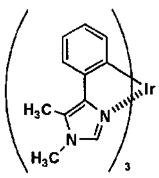
D-42
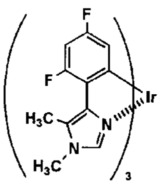

D-43
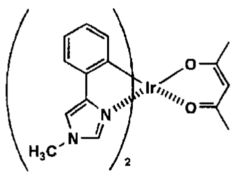
D-44
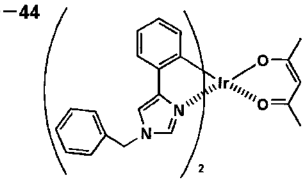

D-45
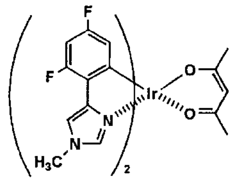
D-46
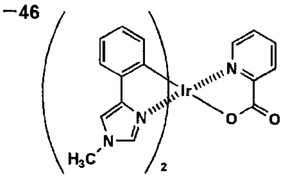

D-47
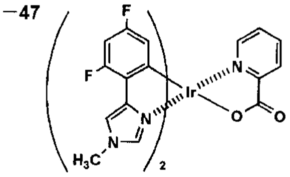
D-48
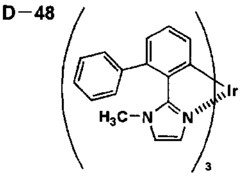

D-49
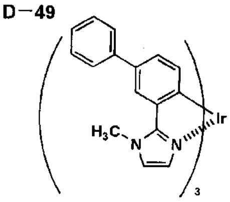
D-50
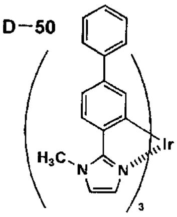
D-51
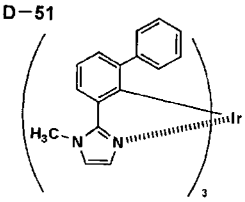

[Chemical Formula 23]

D−52

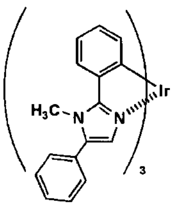

D−53

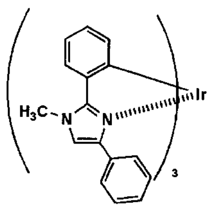

D−54

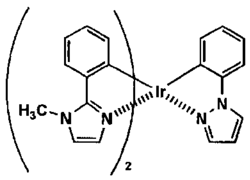

D−55

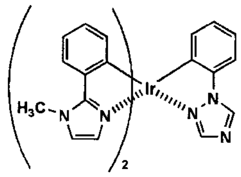

D−56

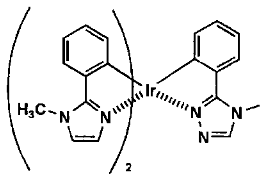

D−57

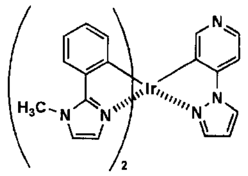

D−58

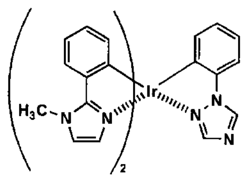

D−59

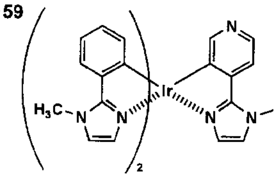

D−60

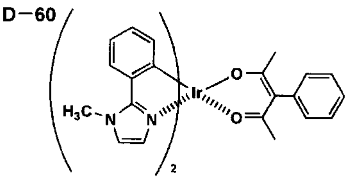

D−61

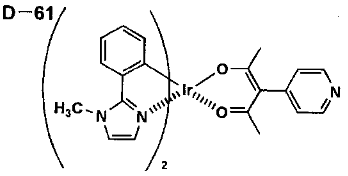

D−62

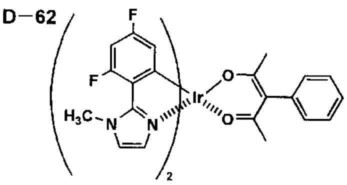

[Chemical Formula 24]

D−63

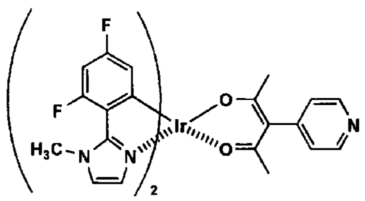

D−64

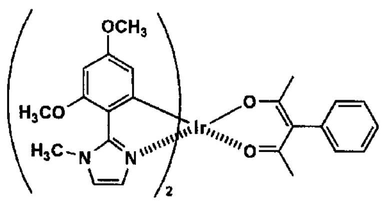

D−65

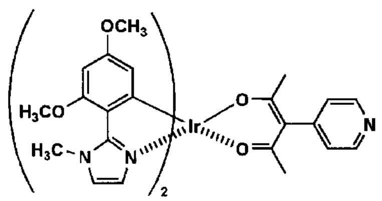

D−66

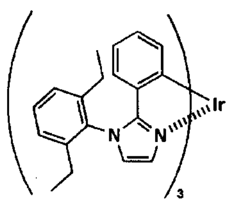

[Chemical Formula 25]

D−67

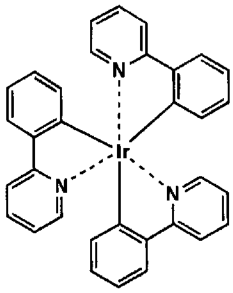

D−68

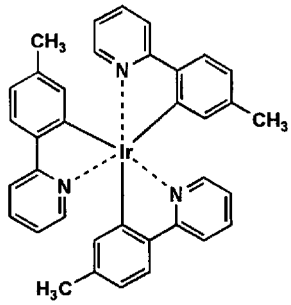

D−69

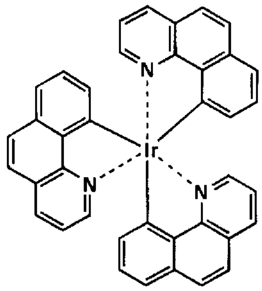

D−70

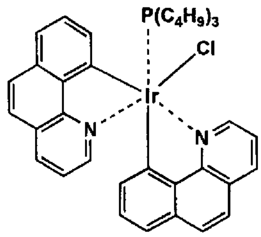

D−71

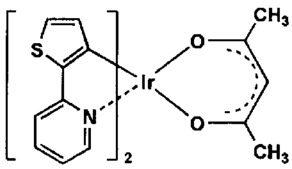

D−72

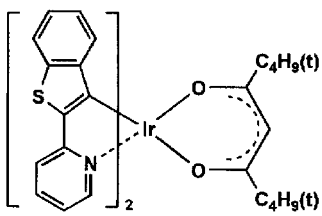

[Chemical Formula 26]

D−73

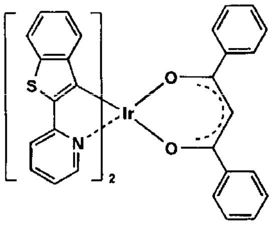

D−74

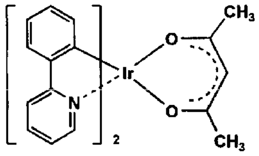

D−75

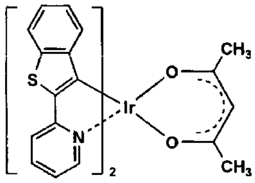

D−76

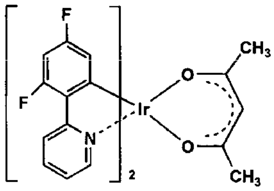

D−77

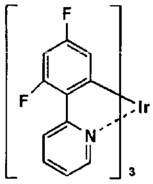

D−78

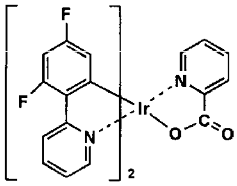

D−79

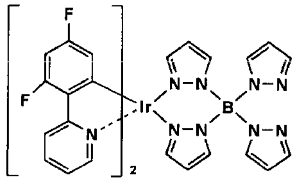

D−80

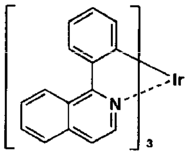

[Chemical Formula 27]

D−81

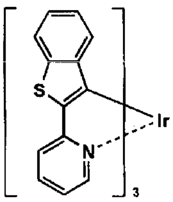

D−82

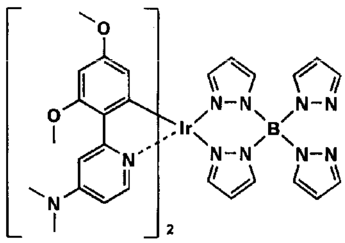

[Chemical Formula 28]

D−83

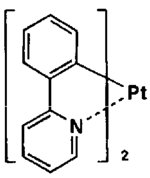

D−84

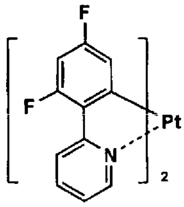

D−85

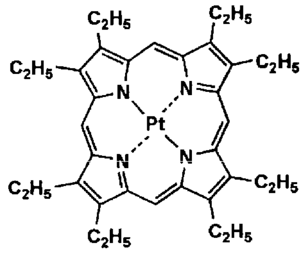

D−86

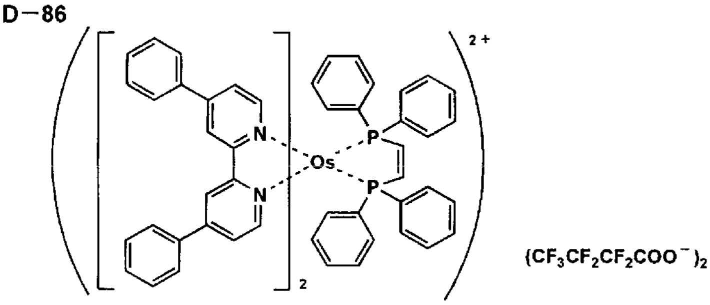

[Chemical Formula 29]

D−87

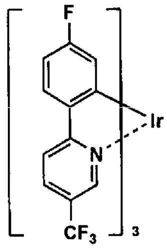

D−88

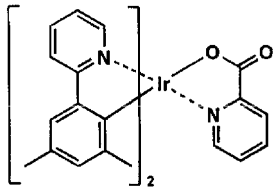

D−89

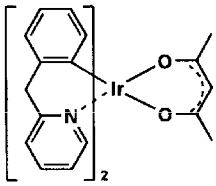

D−90

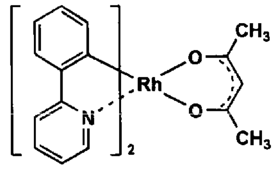

D−91

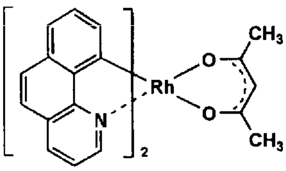

D−92

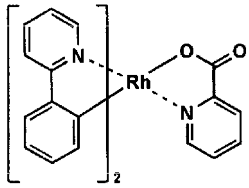

D−93

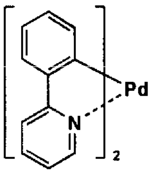

D−94

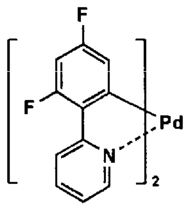

D−95

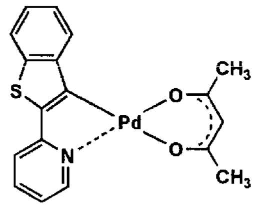

[Chemical Formula 30]

D−96

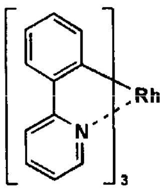

D−97

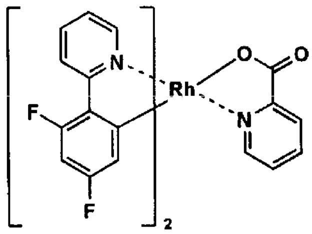

D−98

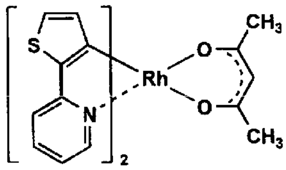

[Chemical Formula 31]

D−99

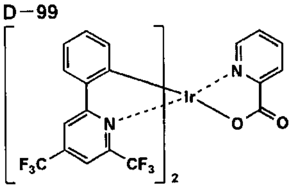

D−100

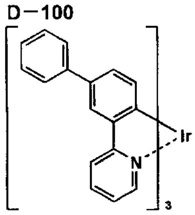

D−101

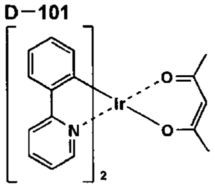

D−102

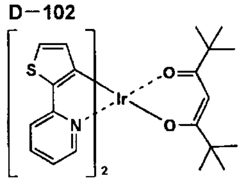

D−103

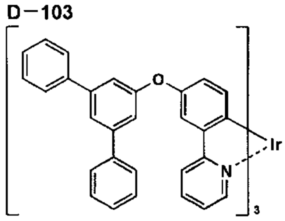

D−104

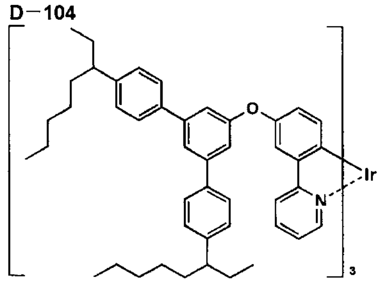

D−105

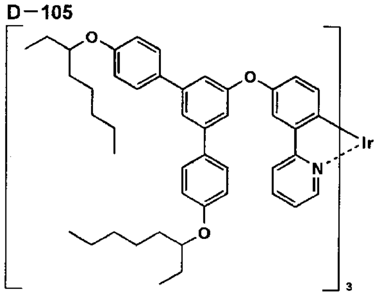

[Chemical Formula 32]

D−106

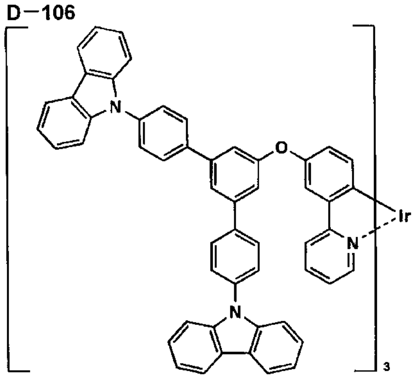

D−107

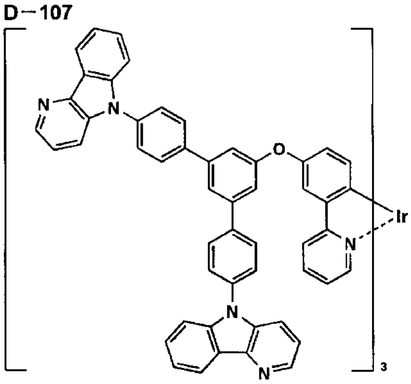

D−108

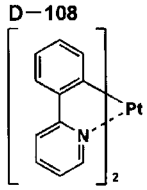

D−109

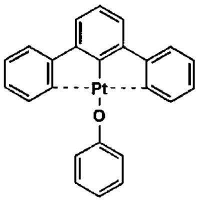

D−110

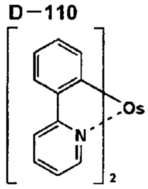

D−111

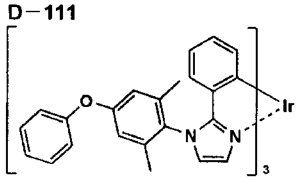

[Chemical Formula 33]

D−112

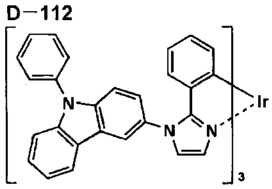

D−113

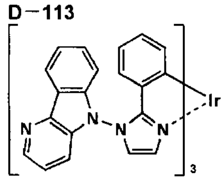

D−114

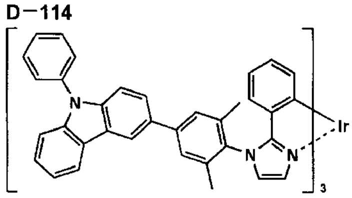

D−115

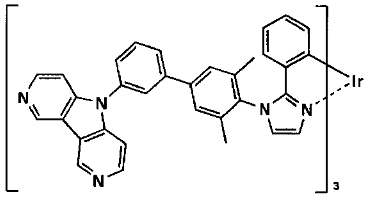

D−116

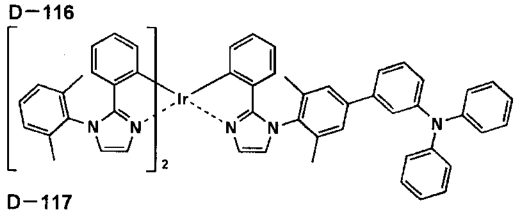

D−117

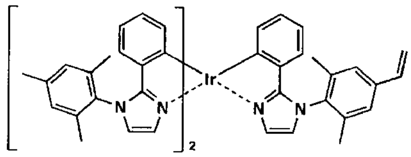

[Chemical Formula 34]

D-118

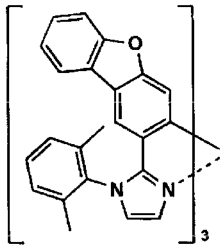

D-119

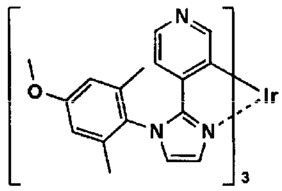

D-120

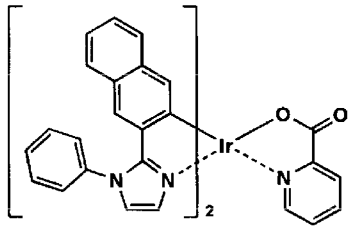

D-121

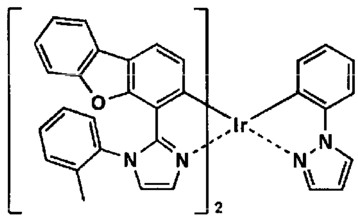

D-122

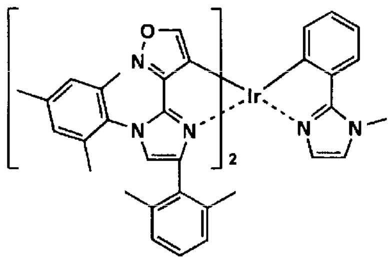

D-123

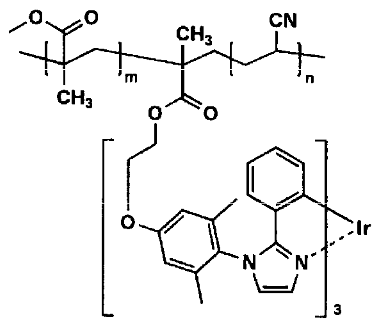

[Chemical Formula 35]

D−124

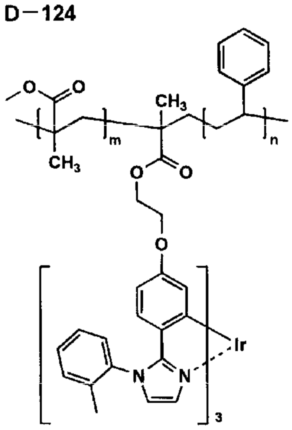

D−125

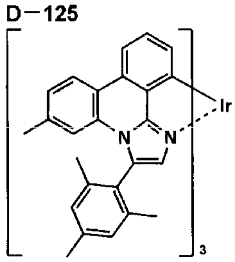

D−126

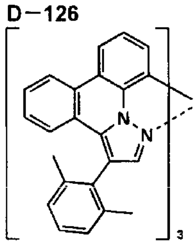

D−127

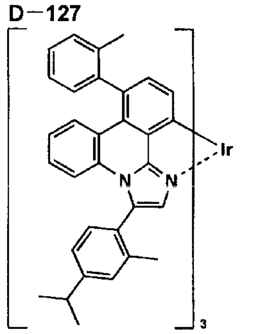

D−128

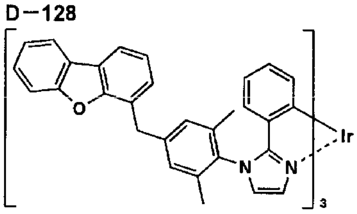

D−129

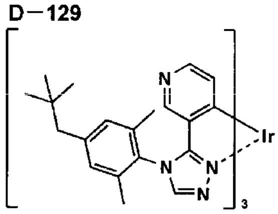

[Chemical Formula 36]

D−130

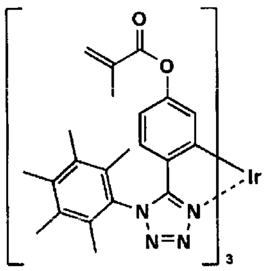

D−131

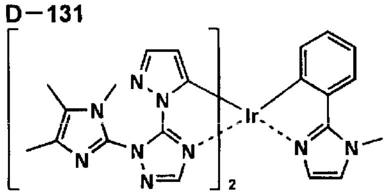

D−132

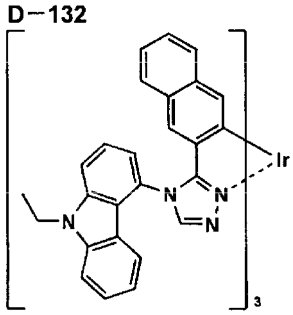

D−133

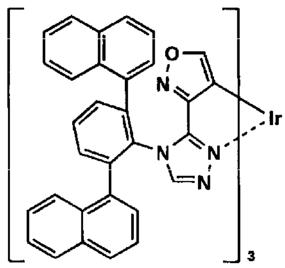

<<Forming Method and Characteristics of Each Organic Functional Layer (Especially Light-Emitting Layer)>>

In the organic electroluminescent element of the present invention, it is enough that at least one light-emitting layer, which is a constituent layer of the element, is formed by an application method. Methods for forming the other layers are not limited to an application method. The other layers may be formed by deposition or the like as needed.

In the method for producing the organic EL element of the present invention, an application method (also referred to as a layer-forming application method) is used as a method for forming the light-emitting layer.

Examples of the method for forming the light-emitting layer include spin coating, casting, ink jetting, spraying, printing, slot die coating and the like.

To obtain a homogeneous layer and to suppress generation of pinholes, layer formation is preferably conducted by an application method such as spin coating, casting, ink jetting, spraying, printing, slot die coating and the like. Among them, slot die coating is more preferable.

It is preferable, of course, to form the constituent layers other than the light-emitting layer of the organic EL element of the present invention by the above application method (layer-forming application method).

After forming the layer, the application liquid is then dried.

Examples of methods for the drying after forming the layer include spin drying, hot-air drying, far-infrared drying, vacuum drying, reduced pressure drying and the like.

(1) Details of Layer-Forming Application Method

Hereinafter, the layer-forming application method for forming the light-emitting layer will be described in detail, as one of the methods for producing the organic EL element of the present invention.

In the present application, the "layer-forming application method" is the method composed of steps from the application of an application liquid to the completion of drying the application liquid.

The present inventors have earnestly studied for suppressing the sizes of fine crystals and their contents in low-molecular organic compound thin layers that were prepared so as to have the same thickness by various layer-forming application methods. The present inventors have then revealed that, by mixing of the multiple kinds of host materials, mixing of multiples kinds of the dopant (light-emitting dopant) materials, mixing of multiple kinds of solvents (light-emitting host), increasing temperature of the application environments and reducing a drying time until the drying decreases the thickness to 5% thicker than the final thickness when the application and the drying are completed, a thin layer can be formed, which thin layer shows a small slope of Guinier plot of scattering radiations obtained by Small-angle X-ray Scattering and derived from the low-molecular organic compounds, that is, a thin layer can be formed, in which thin layer where there is not much aggregation, only a few voids are present and the materials are homogeneously dispersed.

Specifically, the light-emitting layer contains preferably at least two kinds of host materials, more preferably three or more kinds of host materials, further preferably 5 or more kinds of host materials, and further more preferably 7 or more kinds of host materials.

The light-emitting layer contains preferably 3 or more kinds of dopant materials, and more preferably 5 or more kinds of dopant materials.

The application liquid for the light-emitting layer preferably contains a mixed solvent composed of two or more kinds of solvents.

The light-emitting layer is formed preferably at 25 °C or higher, more preferably at 35 °C or higher, and further more preferably at 40 °C or higher.

In the layer-forming method, supercooling may sometimes occur. Thus, cooling may be performed after the application and the drying.

The drying after the layer-forming application may be spin drying, heat-air drying, far-infrared drying, vacuum drying, reduced pressure drying or the like.

In this drying, a drying time is preferably within 10 seconds, more preferably within 5 seconds, further preferably 3 seconds and further more preferably 2 seconds. The drying time is defined as a time from the application of the application liquid through the drying to the timing when the thickness decreases to 5% thicker than the final thickness after the drying.

By the above described ways, a thin layer can be formed, which thin layer shows a small slope of Guinier plot of scattering radiations obtained by Small-angle X-ray Scattering and derived from the low-molecular organic compounds, that is, a thin layer can be formed, in which thin layer where there is not much aggregation, only a few voids are present and the materials are homogeneously dispersed, like a layer formed by deposition.

In the application and the drying of the light-emitting layer or another thin layer, a thin layer is formed upon the application of the liquid, and then its thickness gradually decreases as the drying progresses and the solvent(s) vaporize. When the drying is done thoroughly and the solvent(s) no longer vaporize, the thickness then reaches the final thickness

after the drying.

For example, In the case where the thin layer having the final thickness after the drying of 50 nm is to be formed, the "thickness 5% thicker than the final thickness after the drying" explained above is, for example, 52.5 nm.

The thickness of the light-emitting layer can be measured with a spectroscopic ellipsometer (for example, UVISEL manufactured by JOBIN YVON S.A.S.) or the like.

(2) Characteristics of Light-Emitting Layer

The present inventors have revealed that, in forming the light-emitting layer(s), there exist a preferable molecular weight(s) of, especially, the host(s) (host compound(s)) to form the thin layer which shows a small slope of Guinier plot of scattering radiations obtained by Small-angle X-ray Scattering and derived from the low-molecular organic compounds, that is, to form a thin layer where there is not much aggregation, only a few voids are present and the materials are homogeneously dispersed, like a layer formed by deposition, which influences characteristics of the organic EL element (power efficiency and a life span of light emission).

Specifically, the molecular weight(s) of the low-molecular weight organic compound(s), especially the host(s), in the light-emitting layer(s) are 400 to 2000, and more preferably 400 to 800.

The "molecular weight" in the present description can be obtained by a well-known mass spectroscopy, but is obtained by a well-known GPC (Gel Permeation Chromatography) in the case of a high-molecular weight compound.

Small-angle X-ray scattering of the light-emitting layer may be measured with a multi-purpose apparatus such as a nano-scale X-ray structure characterization apparatus NANO-Viewer manufactured by Rigaku Corporation. Preferably, a Small-angle X-ray scattering apparatus of a large-scaled synchrotron radiation facility such as KEK/PF, Spring8 or SAGA-LS is used. Conditions for the measurement will be described below.

Radiation light of SP-ring8 is used as X-ray, and a thin layer is irradiated with the synchrotron radiation at a wavelength of 1Å.

A multi-axis diffractometer manufactured by HUBER Corporation is used for the measurement. The thin layer sample is irradiated at a fixed incidence angle $\theta$ of 0.2°. A scintillation counter was used as a detector and scattering rays are measured in the range of 1 to 43°.

Data obtained from this Small-angle X-ray scattering are analyzed using particle size and pore size analysis software NANO-Solver manufactured by Rigaku Corporation.

When X-rays enter a substance, some of the X-rays are scattered by electron clouds of the atoms constituting the substance. In small scattering angles (in the present invention, from 1 to 8°), information about spaces in a range of a few to a few hundred nanometer can be obtained. Small-angle X-ray scattering is a structure characterization using this information.

In profiles in Small-angle X-ray scattering, a scattering vector q is used in place of a scattering angle $\theta$, and q is given by the following equation (i):

$$q = (4\pi/\lambda)\sin\theta \ldots (i)$$

In the formula (i), "$\lambda$" represents a wavelength of an X-ray, and "$\theta$" represents a scattering angle.

A region where q is small is called the Guinier region, and a region where q is large is called the Porod region. The former provides information about larger spaces, dispersion state of particles and long period structures etc., and the latter provides information about smaller spaces, polymerization state of high-molecular compounds, shapes of the surfaces of dispersed particles and structural characteristics of proteins etc.

When particles are analyzed using Small-angle X-ray scattering, a Guinier plot is generally used.

If a particle size distribution is relatively narrow and an interaction between particles in a matrix, scattering intensity I(q) is represented by the following equation (ii):

$$I(q) = I(0)\exp(-q^2 * Rg^2/3) \ldots (ii)$$

In the formula (ii), "I(q)" represents scattering intensity, and "Rg" represents a radius of inertia.

This equation is called Guinier Law. When scattering intensity I(q) is plotted with respect to q^2, the slope of the plot depends on radiuses of inertia of scatterers. That is, such a slope includes information about sizes of aggregated clusters with various densities and voids in the layer.

It is known that a slope of a Guinier plot is generally small when scatterers are small and homogeneously dispersed in a layer, and known that a slope of a Guinier plot is large when scatterers are large and eccentrically located.

In the present invention, the above software is used for fitting to a slope of a Guinier plot to obtain a particle size distribution. A distribution ratio of particle sizes is obtained through integrating the particle size distribution.

In an organic electroluminescent element, it is general to form organic layers by deposition in terms of homogeneous dispersibility of molecules and avoidance of impurities in the layers.

However, layer formation by deposition takes time and cost. It is therefore important to obtain a layer with dispersibility similar or higher than that of a deposited layer by an application method being low in cost, for achieving high efficiency and a long life span.

The present inventors have revealed that an organic electroluminescent element with high power efficiency and a long life span can be produced when a distribution curve of the number frequency distribution of the multi-molecular aggregates of molecules of either or both of the dopant and the host in the light-emitting layer has at least one peak and the minimum particle size at a peak top(s) of the peak(s) is 4 nm or less, which distribution curve is obtained from Small-angle X-ray scattering.

The minimum particle size in the distribution curve is preferably 2.5 nm or less, more preferably 2 nm or less, and most preferably 1.5 nm or less.

In the distribution curve of the number frequency distribution of the multi-molecular aggregates of molecules of either or both of the dopant(s) and the host(s), it is preferable that a particle size distribution of the multi-molecular aggregates of molecules of either or both of the dopant(s) and the host(s), which distribution curve is obtained from Small-angle X-ray scattering, also ranges over that minimum value at a peak top (i.e., 1.5 nm) and 15 nm or less. The phrase "a particle size distribution ranges" means that some of the multi-molecular aggregates of molecules of either or both of the dopant(s) and the host(s) are particles having particle sizes ranging over 1.5 nm to 15 nm or less.

In the distribution curve, it is preferable that the particle size distribution ranging over 1.5 nm to 15 nm or less (i.e., the number of the particles having particle sizes of over 1.5 nm to 15 nm or less) accounts preferably for 95% or less, more preferably 70% or less, further preferably 50% or less, further preferably 40% or less, further more preferably 25% or less, and most preferably 25% or less of the total (i.e., the total number of the multi-molecule aggregates of molecules of either or both of the dopant(s) and the host(s)), in terms of achieving high power efficiency and a long life span.

«Anode 2»

For the anode constituting the organic EL element, a metal, alloy, electroconductive compound or a mixture thereof, each of which has a high work function (4 eV or more), is preferably used as an electrode material. Specific examples of the electrode materials include metals such as Au and transparent electroconductive materials such as CuI, indium thin oxide (ITO), $SnO_2$ and ZnO. A material that is amorphous and can be used for a transparent electroconductive layer such as IDIXO ($In_2O_3$-ZnO) may also be used. The anode may be obtained by forming a thin layer with the above-described electrode material(s) by a method such as deposition or sputtering followed by patterning by photolithography to form a desired pattern. In the case where patterning does not need to be so accurate (about 100 μm or more), patterning may be conducted using a mask in a desired shape in deposition or sputtering of the above-described electrode material. In the case of using a compound that is appliable such as an organic electroconductive compound, a layer-forming wet method such as printing or coating may be used. For extracting emitted light from the anode, the transmittance of the anode is desirably 10% or more, and the sheet resistance of the anode is preferably a few hundreds Ω/□ or less. The thickness normally ranges from 10 to 1000 nm, and preferably from 10 to 200 nm, while depending on its material.

«Cathode 8»

On the other hand, for the cathode of the organic EL element of the present invention, a metal, alloy, electroconductive compound or a mixture thereof, each of which has a low work function (4 eV or less) (called an electron-injecting metal) is preferably used as an electrode material. Examples of such an electrode material include sodium, sodium-potassium alloy, magnesium, lithium, a mixture of magnesium and copper, a mixture of magnesium and silver, a mixture of magnesium and aluminum, a mixture of magnesium and indium, a mixture of aluminum and aluminum oxide ($Al_2O_3$), indium, a mixture of lithium and aluminum and rare earth elements. Among them, in terms of electron-injecting properties and resistance against oxidation and the like, a preferable material is a mixture of an electron-injecting metal and a secondary metal that has work function higher than that of the electron-injecting material and is stable, for example, a mixture of magnesium and silver, a mixture of magnesium and aluminum, a mixture of magnesium and indium, a mixture of aluminum and aluminum oxide ($Al_2O_3$), a mixture of lithium and aluminum, aluminum and the like. The cathode may be obtained by forming a thin layer with the above-described electrode material(s) by a method such as deposition, sputtering or the like. Sheet resistance of the cathode is preferably a few hundreds Ω/□ or less, and the thickness of the cathode is normally from 10 nm to 5 μm, and preferably from 50 to 200 nm. To transmit emitted light, it is preferable that the anode or the cathode of the organic EL element is transparent or semi-transparent, which achieves improved

luminance.

The transparent or semi-transparent cathode may be obtained by forming a layer having a thickness of 1 to 20 nm with the above-described metal(s) and subsequently applying the transparent electroconductive material(s) described in the description of the anode on the cathode; by using this procedure, an organic EL element including the anode and the cathode, both of which are transparent, are obtained.

<<Supporting Substrate 1>>

Preferable examples of the transparent supporting substrates (hereinafter also referred to as a base, substrate, base material or support) may be composed of, for example, glass or plastic, but kinds of glasses and plastics are not particularly limited. The supporting substrate 1 may be transparent or opaque. In the case where light is extracted from the side of the supporting substrate, the supporting substrate is preferably transparent. The transparent supporting substrate 1 may be, for example, a glass substrate, a quartz substrate and a transparent resin film. A flexible substrate achieves effects for achieving storage stability in high temperature and suppressing change in chromaticity are achieved more greatly than a rigid substrate does. Thus, a particularly preferable supporting substrate is made from a resin film which is flexible and is capable of providing flexibility for an organic EL element.

Examples of the resin film include films of polyesters such as polyethylene terephthalate (PET) and polyethylene naphthalate (PEN), polyethylene, polypropylene, cellophane, cellulose esters and their derivatives such as cellulose diacetate, cellulose triacetate, cellulose acetate butyrate, cellulose acetate propionate (CAP), cellulose acetate phthalate (TAC) and cellulose nitrate, polyvinylidene chloride, polyvinyl alcohol, polyethylene vinyl alcohol, syndiotactic polystyrene, polycarbonate, norbornene resins, polymethylpentene, polyether ketones, polyimides, polyethersulfone (PES), polyphenylene sulfide, polysulfones, polyether imide, polyether ketone imide, polyamide, fluorine resins, nylon, polymethyl methacrylate, acrylics and polyarylates, and cycloolefin resins such as ARTON (trade name, manufactured by JSR Corp.) and APEL (trade name, manufactured by Mitsui Chemicals Inc.).

On the surface of the resin film, an inorganic or organic coating film or a hybrid coating film composed of the both may be formed. The coating film is preferably a high barrier film having a water vapor transmission rate at 25 ± 0.5°C and 90 ± 2%RH of 0.01 g/(m$^2$·24h) or less determined according to JIS K 7129-1992, and more preferably a high barrier film having an oxygen transmission rate of $10^{-3}$ cm$^3$/(m$^2$·24h·MPa) or less determined according to JIS K 7126-1987 and a water vapor transmission rate of $10^{-3}$ g/(m$^2$·24h) or less, preferably $10^{-5}$ g/(m$^2$·24h) or less.

The barrier film may be formed with any material(s) that can prevent penetration of substances such as moisture and oxygen causing degradation of the element, and usable examples of the materials include silicon oxide, silicon dioxide and silicon nitride. To improve weakness of the film, a barrier film having a laminate structure composed of an inorganic layer and an organic material layer is preferred. The order of these stacked inorganic layer(s) and organic layer(s) is not particularly limited, but it is preferable to stack the inorganic layers and organic layers alternately for multiple times.

The barrier film may be formed by any method without particular limitation. For example, vacuum deposition, sputtering, reactive sputtering, molecular beam epitaxy, ionized-cluster beam method, ion plating, plasma polymerization, atmospheric pressure plasma polymerization, plasma CVD, laser CVD, thermal CVD, or coating may be used, and atmospheric pressure plasma polymerization as described in Japanese Patent Application Laid-Open Publication No. 2004-68143 is particularly preferable.

Examples of the opaque supporting substrate include metal plates such as an aluminum plate and stainless plate, films, opaque resin substrates and ceramic substrates.

In the organic EL element of the present invention, efficiency of external extraction of light at room temperature is preferably 1% or more, and more preferably 5% or more. The efficiency of external extraction of light (%) is obtained by the equation:

efficiency of external extraction of light (%) = the number of photons emitted to the outside of an organic EL element/the number of electrons flowed into the organic EL element × 100

<<Sealing (Sealing Adhesive 10, Sealing member 11)>>

Examples of the sealing ways used in the present invention include a way of bonding a sealing member to the electrode and supporting substrate with an adhesive.

The sealing member is disposed so as to cover a display area composed of the organic EL element(s) and may be in a shape of concave plate or flat plate. The transparency and the electrical insulation properties thereof are not specifically limited.

Specific examples of the sealing members include a glass plate, a composite of polymer plate and film and a composite of metal plate and film. Particular examples of the glass plates include soda-lime grass plates, barium-strontium-containing glass plates, lead glass plates, aluminosilicate glass plates, borosilicate glass plates, barium borosilicate glass plates and quartz plates. Examples of a polymer plate include polycarbonate plates, acrylic plates, polyethylene terephthalate plates, polyethersulfide plates, polysulfone plates. Examples of a metal plate include plates composed of one or more kinds of metals selected from stainless, iron, copper, aluminum, magnesium, nickel, zinc, chromium, titanium, molybdenum, silicon, germanium and tantalum, and plates composed of an alloy(s) of the above metals.

In the present invention, in terms of providing a thin element, polymer films and metal films are preferable. A preferable polymer film has an oxygen transmission rate of $1\times10^{-3}$ $cm^3/(m^2 \cdot 24h \cdot atm)$ or less determined according to JIS K 7126-1987 and a moisture vapor transmission rate at 25 $\pm$ 0.5°C and 90 $\pm$ 2% RH of $1\times10^{-3}$ $g/(m^2 \cdot 24h)$ or less determined according to JIS K 7129-1992.

The sealing member may be made concave by, for example, sandblasting or chemical etching.

The adhesive may be exemplified by light curing or heat curing adhesives containing reactive vinyl groups of an acrylic acid-based oligomer and/or methacrylic acid-based oligomer, moisture curing adhesives such as 2-cyanoacrylate, and heat and chemical curing adhesives (mixture of two kinds of adhesives) such as epoxy adhesives. In addition, hot-melt polyamides, hot-melt polyesters, hot-melt polyolefins, cationic UV curing epoxy resin adhesives may also be given as examples.

To prevent the organic EL element from being deteriorated by heat, preferable adhesives are curable at a temperature ranging from room temperature up to 80°C. In the adhesive, a desiccant may be dispersed. Application of the adhesive to a sealing area may be conducted using a commercially available dispenser or conducted by printing such as screen printing.

It is also preferable to form a layer as a sealing membrane containing an inorganic or organic compound. The sealing membrane is formed on the electrode which sandwiches the organic layer with the supporting substrate so as to cover the electrode and the organic layer and so as to contact to the supporting substrate. A material used for the sealing membrane may be any materials capable of suppressing intrusion of matters that cause deterioration such as water, oxygen and the like. Examples of the material include silicon oxide, silicon dioxide, silicon nitride and the like. To improve weakness of the sealing membrane, the sealing membrane preferably has a laminated structure constituted of the inorganic layer composed of the above inorganic material(s) and an organic layer composed of an organic material(s). The sealing membrane may be formed by vacuum deposition, sputtering, reactive sputtering, molecular beam epitaxy, cluster ion beam, ion plating, plasma polymerization, atmospheric pressure plasma polymerization, plasma CVD, laser CVD, heat CVD or coating, but not specifically limited thereto.

Inert gas such as nitrogen and argon or inert liquid such as fluorohydrocarbon and silicone oil is preferably provided between the sealing member and the display area by injection to provide a gas or liquid medium between the sealing member and a display area composed of the organic EL element(s). The gap between the sealing member and the display area may also be vacuum. Further, a hygroscopic compound may be enclosed within the gap. The hygroscopic compound may be exemplified by metal oxides such as sodium oxide, potassium oxide, calcium oxide, barium oxide, magnesium oxide, aluminum oxide; sulfates such as sodium sulfate, calcium sulfate, magnesium sulfate, cobalt sulfate; metallic halides such as calcium chloride, magnesium chloride, cesium fluoride, tantalum fluoride, cerium bromide, magnesium bromide, barium iodide and magnesium iodide; perchloric acids such as barium perchlorate and magnesium perchlorate. As for sulfates, metallic halides and perchloric acids, their anhydrous salts are preferably used.

Sealing can be either of casing-type sealing and contacting-type sealing. In terms of providing a thin element, contacting-type sealing is preferable. To produce a flexible organic EL element, a sealing member is required to be flexible, and thus contacting-type sealing is preferable.

Preferred embodiments for the contacting-type sealing will now be described.

The sealing adhesive used in the present invention may be, for example, a heat curable adhesive or UV curable resin, preferably a heat curable adhesive such as epoxy resin, acrylic resin or silicone resin, and more preferably an epoxy heat curable adhering resin which has excellent humid and water resistance and causes small shrinkage in curing.

The moisture content in the sealing adhesive used in the present invention is preferably 300 ppm or less, more preferably 0.01 to 200 ppm, and most preferably 0.01 to 100 ppm.

The moisture content referred herein may be measured by any method, for example, by using a volumetric titrator (Karl Fischer), an infrared moisture gauge, a microwave transmission moisture gauge, heating and drying gravimetry, GC/MS, IR, differential scanning calorimeter (DSC) or thermal desorption spectroscopy (TDS). The moisture content of a film, solid film or the like may be obtained with rise in pressure caused by water evaporation using a precision moisture meter AVM-3000 manufactured by OMNITEK.

In the present invention, the moisture content of the sealing adhesive may be controlled by putting the sealing adhesive into a nitrogen atmosphere wherein the dew point is -80°C or less and the oxygen concentration is 0.08 ppm for a period adequately varied, or by drying the sealing adhesive by putting the sealing adhesive into vacuum at a

pressure of less than 100 Pa for a period adequately varied. The sealing adhesive may be dried alone, or may be disposed on the sealing member and then dried together.

In the case of contacting-type sealing, as the sealing member, polyethylene terephthalate (PET) having a thickness of 50 μm on which an aluminum film having a thickness of 30 μm is formed is used. On the aluminum side of this sealing member, the sealing adhesive is uniformly applied using a dispenser, and then the resin substrate 1 is positioned with the sealing member 5. Thereafter, pressure bonding of the resin substrate 1 and the sealing member 5 is conducted (at a pressure of 0.1 to 3 MPa) and the resin substrate 1 and the sealing member 5 are adhered to each other at 80 to 180°C. Contacting-type sealing is thus conducted.

Periods for heating and pressure bonding vary according to, for example, a kind(s) of adhesives, the amount of adhesive or an area for sealing, but heating and pressure bonding may be conducted in conditions of temporary adhesion at a pressure of 0.1 to 3 MPa, heating at a temperature of 80 to 180°C and heat curing for 5 seconds to 10 minutes.

It is preferable to use a heated pressure bonding roll because both heating and pressure bonding (temporary adhesion) can be simultaneously conducted and inner voids can be eliminated.

The layer of the adhesive may be formed by a coating method such as roll coating, spin coating, screen printing or spray coating, or printing, optionally using a dispenser according to a kind(s) of materials.

Contacting-type sealing is covering with a cured resin causing no void between a sealing member and a substrate of an organic EL element. Examples of the sealing member include metals such as stainless, aluminum and magnesium alloy, plastics such as polyethylene terephthalate, polycarbonate, polystylene, nylon and polyvinyl chloride, composites thereof and glass. When needed, particularly in the case of using a resin film, a sealing member on which a gas barrier layer composed of aluminum, aluminum oxide, silicon oxide or silicon nitride is formed can be used, like the resin substrate described above. The gas barrier layer may be formed on the both or one surface of a sealing member by sputtering, deposition or the like before shaping the sealing member, or may be formed on the both or one surface of a sealing member by a method such as the above after sealing. This gas barrier layer preferably has an oxygen transmission rate of $1\times10^{-3}$ ml/($m^2\cdot$24h·atm) or less and a water vapor transmission rate at 25 ± 0.5 °C and 90 ± 2% RH of $1\times10^{-3}$ g/($m^2\cdot$24h) or less.

The sealing member may be a film on which a metal foil such as an aluminum foil is provided. To form a polymer film on one side of a metal foil, a commonly-used laminating device may be used. An adhesive may be a polyurethane adhesive, polyester adhesive, epoxy adhesive or acrylic adhesive. A curing agent may be used in combination with the adhesive as needed. Lamination may be conducted by hot-melt lamination, extrusion lamination or co-extrusion lamination, and dry lamination is preferable.

When a metal foil is formed by sputtering, deposition or the like with a fluid electrode material such as an electroconductive paste, the sealing member may formed by a way that is reverse to the above, namely, by forming a metal foil on a substrate of polymer film.

«Protective Film, Protective Plate»

A protective film or protective plate may be provided on the other side of the sealing membrane or sealing film, either of which is provided on the side sandwiching the organic functional layer(s) with the supporting substrate, in order to improve mechanical strength of the organic EL element. It is preferable to provide the protective film or protective plate especially in the case of sealing with the sealing membrane because the sealing membrane is not so mechanically strong. Materials for the protective film or protective plate may be exemplified by a glass plate, a composite of polymer plate and film and a composite of metal plate and film, like the materials for sealing. To achieve light weight and thinness, polymer film is preferable.

In the present invention, it is preferable to provide a light-extracting member between the flexible supporting substrate and the anode, or at any position from the flexible supporting substrate to a side from which light is extracted.

The light-extracting member may be exemplified by a prism sheet, lens sheet and diffusing sheet, and may also be exemplified by a diffracting grating or a diffusing structure provided in any medium or at an interface where total reflection occurs. Generally in the case of light emission from the side of a substrate of an organic electroluminescent element, part of light emitted from a light-emitting layer is totally reflected at the interface between a substrate and the air, resulted in loss of light. To solve this problem, the surface of the substrate is treated to form prism or lens structures, or a prism sheet, lens sheet or diffusing sheet is pasted on the surface of the substrate. Accordingly, total reflection is suppressed and efficiency of light extraction is improved.

To improve efficiency of light extraction, there is a known method for introducing a diffraction grating or a diffusing structure in any medium or at an interface where total reflection occurs.

«Method for Manufacturing Organic EL Element 100»

As an example of the method for manufacturing the organic EL element of the present invention, a method for

manufacturing an organic EL element composed of an anode/hole-injecting layer/hole-transporting layer/light-emitting layer/electron-transporting layer/electron-injecting layer/cathode will now be described.

The anode is obtained by forming a thin layer having a thickness of 1 μm or less, preferably 10 to 200 nm and composed of an intended electrode material(s), for example, a material(s) for the anode on a suitable substrate.

Subsequently, the organic functional layers (organic compound thin layers) of the organic EL element, i.e., the hole-injecting layer, the hole-transporting layer, the light-emitting layer, the electron-transporting layer and the electron-injecting layer, are formed on/over the anode.

Formation of the organic functional layers is mainly composed of the steps of:

(i) application and stacking of an application liquid for each organic functional layer on/over the anode on the supporting substrate; and
(ii) drying of each applied and stacked application liquid.

A method for forming the light-emitting layer (a layer-forming application method) is such as described above.

In the step (i), a method for forming each layer may be deposition and a wet process (such as spin coating, casting, die coating, blade coating, roll coating, ink jetting, printing, spray coating, curtain coating, Langmuir Blodgett (LB) method). Preferably, at least the light-emitting layer of the present invention is formed by a wet process.

In forming layers of the organic functional layers other than the hole-injecting layer, a wet process is preferable for the present invention, because homogeneous layers can be formed and pinholes are hardly formed, for example. Particularly, the layers are preferably formed by an application method such as spin coating, casting, die coating, blade coating, roll coating and ink jetting.

Examples of a liquid medium dissolving or dispersing a material(s) for the organic EL element therein include ketones such as methylethyl ketone and cyclohexanone; aliphatic acid esters such as ethyl acetate; halogenated hydrocarbons such as dichlorobenzene; aromatic hydrocarbons such as toluene, xylene, mesitylene and cyclohexylbenzene; aliphatic hydrocarbons such as cyclohexane, decaline and dodecane; and organic solvents such as dimethyl formamide (DMF) and dimethyl sulfoxide (DMSO). A method for dispersion may be, for example, ultrasonic wave dispersion, high shearing force dispersion or medium dispersion.

Processes from a preparation step by dissolving or dispersing a material(s) for the organic EL element of the present invention in a liquid medium to the application step of the liquid on/over the substrate are preferably conducted in an inert gas atmosphere. However, in the case of using certain kinds of materials, layers can be formed in a non-inert gas atmosphere without decreasing performances of an organic EL element, and therefore using an inert gas atmosphere is not necessary. Such a case is preferably because production cost can be reduced.

In the step (ii), each of the organic functional layers formed by application and stacking is dried. This drying means that the content of the solvent in a formed layer decreases by 0.2% or less defining the content of the solvent of the formed layer immediately after the application as 100%.

The drying can be conducted by a well-known method such as reduced pressure drying, press drying, heat drying, blow drying, IR drying and drying using electromagnetic rays. Among them, heat drying is preferable. It is most preferable that the drying temperature is kept at (Tg + 20)°C or less wherein the Tg is the lowers Tg among Tgs of materials of the organic functional layer. Specifically, in the present invention, the drying is preferably conducted at a temperature kept in the range from 80 to 150°C, and more preferably at 100 to 130°C.

It is preferable that an atmosphere in the drying the applied and stacked application liquid has a volume concentration of a gas(es) other than an inert gas(es) of 200 ppm or less. As in the preparation and application steps, the drying can be conducted in a non-inert gas atmosphere in certain cases. Such cases are preferable because production cost can be reduced.

The inert gas is preferably a noble gas such as nitrogen gas and argon gas. In view of production cost, nitrogen gas is preferable.

The steps of application, stacking and drying may be conducted by a single wafer production system or line production system. The drying step may be conducted during conveyance on the line. In terms of productivity, the drying step may be conducted after stacking or non-contact winding to form a roll.

After forming these layers, a thin layer composed of the material for the cathode is formed thereon so as to have a thickness of 1 μm or less, preferably a thickness of 50 to 200 nm on the outermost layer by a method such as vapor deposition or sputtering as the cathode. An intended organic EL element is thus produced.

After the heating, contacting-type sealing or bonding of the sealing member to the electrodes and the supporting substrate with the adhesive is conducted, and then the organic EL element can be obtained.

«Uses»

The organic EL element of the present invention may be used for display devices, displays and various light

sources (or illumination devices).

There are various examples of the light sources. For example, the light source can be a household lighting, an in-car lighting, a backlight of a clock or liquid crystal display, a billboard, a traffic signal, a light source of an optical storage medium, a light source of an electro photocopier, a light source of an optical communication processer, a light source of an optical sensor and a general electric home appliance which requires a display device. Particularly, the organic element of the present invention may be effectively used for a backlight of a liquid crystal display device combined with a color filter and a light source for illumination.

In the organic EL element of the present invention, patterning may be conducted in forming a layer using a metal mask or by inkjet printing method or the like as needed. Patterning may be conducted only on the electrode(s), on the electrodes and the light-emitting layer, or on all of the layers of the element. In manufacturing the element, any conventionally known method(s) may be used.

## Examples

The present invention will now be described in detail with reference to Examples, but is not limited thereto. In Examples, "part(s)" and "%" means "part(s) by mass" and "% by mass" unless described otherwise.

### «Production of Organic EL Element»

### (1) Production of Example Sample 1

#### (1.1) Preparation of Gas-Barrier Flexible Film

As a flexible film, polyethylene naphthalate film (manufactured by Teijin DuPont Films Japan Limited, hereinafter abbreviated as PEN) was used. On its entire surface of the side on which a first electrode was to be provided, a 500 nm-thick gas barrier film was seamlessly formed using an inorganic compound composed of SiOx with an atmospheric pressure plasma discharge processor configured as described in Japanese Patent Application Laid-open Publication No.2004-68143. A flexible gas barrier film having an oxygen transmission rate of 0.001 ml/$m^2$/day or less and a water vapor transmission rate of 0.001 g/$m^2$/day or less was thus prepared.

#### (1.2) Formation of First Electrode

On the prepared bass barrier flexible film, a layer having a thickness of 120 nm was formed using ITO (indium tin oxide) by sputtering followed by patterning by photolithography. A first electrode (anode) was thus formed.

The formed pattern had a light-emitting area of 50 square millimeter.

#### (1.3) Formation of Hole-Injecting Layer

The patterned ITO substrate was washed by ultrasonic washing with isopropyl alcohol, dried in a dry nitrogen atmosphere and washed by UV ozone washing for 5 minutes. On the resulting substrate, a layer was formed using a 70% solution of poly(3,4-ethylenedioxythiophene)-polystylene sulfonate (abbreviated as PEDOT/PSS, P AI 4083 manufactured by Bayer AG) in pure water by spin coating at 3000 rpm for 30 seconds, and then drying was conducted at 200°C for an hour. A hole-injecting layer having a thickness of 30 nm was thus formed.

#### (1.4) Formation of Hole-Transporting Layer

The resulting substrate was then put in a nitrogen atmosphere using nitrogen gas (G1 Grade), and a layer was formed thereon with a 0.5% solution of the exemplary compound (60) (Mw = 80,000), which is an electron hole-transporting material, in chlorobenzene by spin coating at 1500 rpm for 30 seconds, and the layer was kept at 130°C for 30 minutes. A hole-transporting layer having a thickness of 30 nm was thus formed.

#### (1.5) Formation of Light-Emitting Layer

A layer was then formed using respective compounds below in the following composition ratios for a light-emitting layer by spin coating at 1500 rpm for 30 seconds, and the layer was then kept at 120 °C for 30 minutes. A light-emitting layer having a thickness of 40 nm was thus formed.

<Compounds for Light-Emitting Layer>

Host material ... Mixture of equal masses of the exemplary compounds a-1, a-2, a-6, a-7, a-20, a-27, a-38 and a-41 13.95 parts
Dopant material ... Mixture of equal masses of the exemplary compounds D-1, D-2, D-5, D-28 and D-66 2.45 parts
Dopant material ... Exemplary compound D-67 0.025 part
Dopant material ... Exemplary compound D-80 0.025 part
Solvent ... Toluene : isopropyl acetate = 1:1 2,000 parts
During the application, ambient temperature was kept at 40°C and the applied liquid was aired with dry air.

(1.6) Formation of Electron-Transporting Layer

Next, a layer was formed using a solution in which 20 mg of the exemplary compound A, which is represented by the general formula A, was dissolved in 4 ml of tetrafluoro propanol (TFPO), by spin coating at 1500 rpm for 30 seconds, and the layer was then kept at 120°C for 30 minutes. An electron-transporting layer having a thickness of 30 nm was thus formed.

[Chemical Formula 37]
Compound A

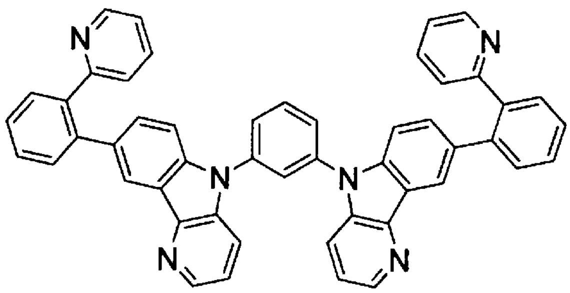

(1.7) Formation of Electron-Injecting Layer and Cathode

Thereafter, the resulting substrate was put in a vacuum deposition device without being exposed to the air. A molybdenum resistive heating boat in which sodium fluoride was placed and a molybdenum resistive heating boat in which potassium fluoride was placed were also put in the vacuum deposition device. Then its vacuum chamber was depressurized by $4\times10^{-5}$ Pa, and the boat was electrified to be heated so as to form a thin layer having a thickness of 1 nm with sodium fluoride at a rate of 0.02 nm/sec on the electron-transporting layer, and by the same way, an electron-injecting layer having a thickness of 1.5 nm was formed on the sodium fluoride layer at a rate of 0.02 nm/sec.

Thereafter, aluminum was vapor-deposited so as to form a cathode having a thickness of 100 nm.

(1.8) Sealing and Production of Organic EL Element

Subsequently, a sealing member was adhered to the resulting substrate using a commercially-available roll lamination device to produce Example Sample 1 (the organic EL element).

Specifically, as the sealing member, a 30 μm-thick flexible aluminum foil (manufactured by TOYO ALUMINIUM K.K.) on which a 12 μm-thick polyethylene terephthalate film was formed using an adhesive for dry lamination so as to obtain a thickness of the adhesive layer of 1.5 μm, which adhesive is a two-liquid reaction type urethane adhesive, was used.

On a side for adhesion (glossy side) of the aluminum foil, a heat curing adhesive as the sealing adhesive was evenly applied using a dispenser to obtain a thickness of 20 μm, followed by drying in vacuum at a pressure of 100 Pa or less for 12 hours.

Thereafter, the sealing member was put in a nitrogen atmosphere wherein the dew point was -80°C and the oxygen concentration was 0.8 ppm and was dried for not less than 12 hours so as to adjust the moisture content in the sealing adhesive to 100 ppm or less.

The heat curing adhesive was an epoxy adhesive composed of a mixture of the following (A) to (C).

(A) bisphenol A diglycidyl ether (DGEBA)
(B) dicyandiamide (DICY)
(C) epoxy adduct curing accelerator

As described above, the resulting substrate to be sealed was arranged on and adhered to an extracting electrode and a junction(s) of an electrode lead(s) so as to cover the extracting electrode and the junction(s) of the electrode lead(s), and the contacting-type sealing was conducted in the condition that a temperature of a pressure bonding roll was 120 °C, pressure of the bonding was 0.5 MPa and a rate of the device of 0.3 m/minute to form an element as illustrated in Fig. 1. Example Sample 1 (the organic EL element) was thus produced.

(2) Production of Example Samples 2 to 5

Example Samples 2 to 5 were prepared by the same way as Example Sample 1 was prepared except that D-1 and D-2 out of the above dopant materials were not used and the conditions for the application and the drying (solvent species, temperatures in the application, use or non-use of dry air and drying times) were changed as shown in Table 1.

(3) Production of Example Sample 6

Example Sample 6 was prepared by the same way as Example Sample 1 was prepared except that a-20, a-27 and a-38 out of the above host materials were not used, D-1 and D-2 out of the above dopant materials were not used and the conditions for the application and the drying were changed as shown in Table 1.

(4) Production of Example Sample 7

Example Sample 7 was prepared by the same way as Example Sample 1 was prepared except that a-6, a-7, a-20, a-27 and a-38 out of the above host materials were not used, D-1 and D-2 out of the above dopant materials were not used and the conditions for the application and the drying were changed as shown in Table 1.

(5) Production of Example Samples 8 and 9

Example Samples 8 and 9 were prepared by the same way as Example Sample 1 was prepared except that a-2, a-6, a-7, a-20, a-27 and a-38 out of the above host materials were not used, D-1 and D-2 out of the above dopant materials were not used and the conditions for the application and the drying were changed as shown in Table 1.

(6) Production of Comparative Example Sample 10

Comparative Example Sample 10 was prepared by the same way as Example Sample 1 was prepared except that the materials for the light-emitting layer were changed as described below and the conditions for the application and the drying were changed as shown in Table 1.

<Compounds for Light-Emitting Layer>

| | |
|---|---|
| Host Materials ... the exemplary compounds a-38 and a-41 | 13.95 parts |
| Dopant material ... the exemplary compound D-66 | 2.45 parts |
| Dopant material ... the exemplary compound D-67 | 0.025 part |
| Dopant material ... the exemplary compound D-80 | 0.025 part |
| Solvent ... Toluene | 2,000 parts |

(7) Production of Comparative Example Samples 11 to 13

Comparative Example Samples 11 to 13 were prepared by the same way as Example Sample 1 was prepared except that the materials for the light-emitting layers were changed as described below and the conditions for the application and the drying were changed as shown in Table 1.

<Compounds for Light-Emitting Layer>

| | |
|---|---|
| Host Material ... the exemplary compound a-38 | 13.95 parts |
| Dopant material ... the exemplary compound D-66 | 2.45 parts |
| Dopant material ... the exemplary compound D-67 | 0.025 part |
| Dopant material ... the exemplary compound D-80 | 0.025 part |
| Solvent ... Toluene | 2,000 parts |

[Table 1]

| SAMPLE No. | NUMBER OF MIXED HOSTS (KINDS) | NUMBER OF MIXED DOPANTS (KINDS) | APPLICATION CONDITIONS | | | |
|---|---|---|---|---|---|---|
| | | | KIND(S) OF SOLVENT(S) | TEMPERATURE [C°] | DRY AIR | DRYING TIME [SEC] |
| 1 | 8 | 7 | TOLUENE : ISOPROPYL ACETATE = 1:1 | 40 | USED | 1.8 |
| 2 | 8 | 5 | | | | |
| 3 | | | TOLUENE | 40 | USED | 2 |
| 4 | | | | 25 | | 3 |
| 5 | | | | 40 | NOT USED | 10 |
| 6 | 5 | 5 | TOLUENE : ISOPROPYL ACETATE = 1:1 | 40 | USED | 1.8 |
| 7 | 3 | 5 | | | | 2.2 |
| 8 | 2 | 5 | | | | 2.4 |
| 9 | | | TOLUENE | 40 | USED | 5 |
| 10 | 2 | 3 | TOLUENE | 40 | USED | 8 |
| 11 | 1 | 3 | TOLUENE | 40 | USED | 13 |
| 12 | | | | 25 | | 15 |
| 13 | | | | | NOT USED | 18 |

The compositions and conditions for the application and drying of the above Example Samples 1 to 9 and Comparative Example Samples 10 to 13 are shown in Table 1.

<<Evaluation of Organic EL Elements>>

As to Example Samples 1 to 9 and Comparative Example Samples 10 to 13, (1) their particle size distributions were obtained by analyzing data from Small angle X-ray scattering, and the evaluations (2) to (6) were conducted.

(1) Particle Size Distribution Analysis of Results from Small-angle X-Ray Scattering

The light-emitting layers of Samples 10 to 13 were each formed as thin layers on silicon wafers to prepare samples to be subjected to the measurement. X-ray used was synchrotron radiation of SPring-8, and the thin layer samples were radiated with the synchrotron radiation at a wavelength of 1Å. In the measurement, the thin layer samples were irradiated at a fixed incidence angle θ of 0.2°. A scintillation counter was used as a detector and the measurement

was conducted in the range of 1 to 43°. Data obtained from scattering diffusion was used to draw particle size distribution curves with the previously described software (see Fig. 2).

Results of the analysis is shown in Table 2.

In Table 2, "minimum particle size" means the minimum particle size at a peak top(s) of a peak(s) in the particle size distribution curve, and "ratio of integral values of particle size distribution" means, in principle, a ratio of the number of the particles having particle sizes over 1.5 nm to 15 nm or less to the total number of the particles, the particles being the multi-molecular aggregates of molecules of either or both of the dopant(s) and the host(s).

(2) Measurement of Luminance and Driving Voltage

Each of Samples was made emit light at room temperature (23 to 25 °C) at a constant luminance of 1,000 cd/$m^2$ and then subjected to the measurement of luminance with a spectroradiometer CS-2000 (manufactured by Konica Minolta Sensing Inc.). The luminance (at a constant current) and driving voltage at a luminance of 1000 cd/$m^2$ were thus obtained. Results of the measurement are shown in Table 2. In Table 2, the luminance and driving voltages of Samples 1 to 10 and 12 to 13 are indicated by relative values defining the luminance and driving voltage of Sample 11 (Comparative Example) as "100".

(3) Evaluation of Stability against Continuous Driving (Life Span)

Each of Samples was put on a cylinder with a radius of 5 cm so as to bend the Sample, and the bent Sample was continuously driven. Luminance of each bent Sample was measured with the above spectroradiometer CS-2000. A time period until the luminance decreased by half (LT50) was obtained. In the driving, the current value was determined so that the luminance was 4000 cd/$m^2$ at the start of the continuous driving.

Relative values of LT50s were calculated defining the LT50 of Sample 11 (Comparative Example) as 100. The relative values were indicators of stabilities in continuous driving. When the relative value is high, it means that the Sample having such a value is excellent in stability in a continuous driving (has a longer life span).

(4) Evaluation of Properties in Application

Each of Samples were made emit light at room temperature (23 to 25°C) at a constant luminance of 1,000 cd/$m^2$ and then subjected to visual estimation of unevenness of light emission. The unevenness was ranked into four levels according to the following criteria.

"◎ (double circle)": no unevenness was observed
"○ (single circle)": unevenness was slightly observed
"Δ (triangle)": unevenness was observed but unobservable when seen from 0.3 m away or further
"× (cross)": unevenness was observed even when seen from 0.3 m away or further

(5) Calculation of Yield Rate

For each Sample, 10 samples were produced. These samples were made emit light at room temperature (23 to 25 ° C) at a constant luminance of 1,000 cd/$m^2$ and then subjected to the examination of the presence or absence of points that do not emit light (i.e., dark spots).

For each Sample, the light-emitting element thereof was ideally divided into 5 by 5 blocks. The block(s) in which a dark spot(s) were found were defined as a defective block(s). The rate of the defective block(s) to the total 250 blocks (i.e., the yield rate) was calculated. Results of the examination are shown in Table 2.

(6) Evaluation of Stability in Storage at High Temperature

Each of the organic EL elements was stored at 85 °C for 300 hours. Luminance before this high-temperature treatment and luminance after this high-temperature treatment were measured with a spectroradiometer CS-2000 (manufactured by Konica Minolta Sensing Inc.). As a rate of change in luminance, Δluminance (%) was obtained by the equation below to be an indicator of stability in storage at high temperature. Results of the evaluation are shown in Table 2.

It is indicated that the element having a small Δluminance (%) is excellent in stability in storage at high temperature.

$$\Delta \text{luminance} (\%) = \{(\text{luminance before the high-temperature treatment}) - (\text{luminance after the high-temperature treatment}) / \text{luminance before the high-temperature treatment}\} \times 100$$

[Table 2]

| SAMPLE No. | MINIMUM PARTICLE SIZE [nm] | RATIO OF INTEGRAL VALUES OF PARTICLE SIZE DISTRIBUTION (NUMBER OF 1.5 nm LARGE PARTICLES/ TOTAL NUMBER OF PARTICLES) | EVALUATION RESULTS | | | | | | NOTE |
|---|---|---|---|---|---|---|---|---|---|
| | | | LIGHT EMISSION EFFICIENCY | | LIGHT EMISSION LIFE SPAN | PROPERTIES IN APPLICATION | YIELD RATE [%] | STABILITY IN STORAGE [%] | |
| | | | LUMINANCE | DRIVING | | | | | |
| 1 | 0.8 | 20% | 140 | 69 | 215 | ◎ | 92 | 5 | PRESENT INVENTION |
| 2 | 1 | 25% | 135 | 72 | 205 | ◎ | 91 | 7 | |
| 3 | 1.2 | 29% | 135 | 73 | 200 | ◎ | 91 | 10 | |
| 4 | 1.3 | 40% | 135 | 75 | 198 | ◎ | 91 | 12 | |
| 5 | 1.3 | 45% | 125 | 76 | 191 | ○ | 87 | 14 | |
| 6 | 1.5 | 55% | 128 | 78 | 183 | ○ | 90 | 15 | |
| 7 | 2 | - | 120 | 81 | 164 | ○ | 85 | 18 | |
| 8 | 2.6 | - | 117 | 87 | 148 | Δ | 83 | 20 | |
| 9 | 3.9 | - | 110 | 94 | 120 | Δ | 80 | 25 | |
| 10 | 4.2 | - | 101 | 100 | 102 | Δ | 69 | 37 | COMPARATIVE EXAMPLE |
| 11 | 5.1 | - | 100 | 100 | 100 | Δ | 68 | 42 | |
| 12 | 5.4 | - | 98 | 101 | 97 | Δ | 63 | 50 | |
| 13 | 5.9 | - | 96 | 104 | 93 | × | 60 | 58 | |

(7) Conclusion

As demonstrated in Table 2. Example Samples 1 to 9 show excellent results for all of the evaluations compared to Comparative Examples 10 to 13.

Therefore, to improve at least efficiency of light emission and a life span of light emission, it is proved to be effective that a distribution curve of the number frequency distribution of the multi-molecular aggregates of molecules of either or both of the dopant(s) and the host(s) has at least one peak and the minimum particle size at a peak top(s) of the peak(s) is 4 nm or less, which distribution curve is obtained from Small-angle X-ray scattering performed on the light-emitting layer.

Industrial Applicability

The present invention can be suitably used for providing an organic EL element with both of highly-efficient light emission and a long life span.

Description of Reference Numerals

| | |
|---|---|
| 1 | Flexible Supporting Substrate |
| 2 | Anode |
| 3 | Hole-Injecting Layer |
| 4 | Hole-Transporting Layer |
| 5 | Light-Emitting Layer |
| 6 | Electron-Transporting Layer |
| 7 | Electron-Injecting Layer |
| 8 | Cathode |
| 9 | Sealing Adhesive |
| 10 | Flexible Sealing Member |
| 20 | Organic Functional Layers |
| 100 | Organic Electroluminescence Element |

## Claims

**1.** An organic electroluminescent element comprising, on/over a substrate:

a pair of electrodes; and
at least one light-emitting layer, wherein
the light-emitting layer contains at least one kind of dopant and at least one kind of host,
the light-emitting layer is formed by an application method using an application liquid containing at least one kind of solvent,
a distribution curve of a number frequency distribution of multi-molecular aggregates of either or both of molecules of the dopant and the host in the light-emitting layer has at least one peak, which distribution curve is obtained from Small-angle X-ray scattering, and
a minimum particle size at peak top(s) of the peak(s) is 4 nm or less.

**2.** An organic electroluminescent element comprising, on/over a substrate:

a pair of electrodes; and
at least one light-emitting layer, wherein
the light-emitting layer contains at least one kind of dopant and at least one kind of host,
the light-emitting layer is formed by an application method using an application liquid containing at least one kind of solvent,
a distribution curve of a number frequency distribution of multi-molecular aggregates of either or both of molecules of the dopant and the host in the light-emitting layer has at least one peak, which distribution curve is obtained from Small-angle X-ray scattering, and
a minimum particle size at peak top(s) of the peak(s) is 2.5 nm or less.

3. An organic electroluminescent element comprising, on/over a substrate:

a pair of electrodes; and
at least one light-emitting layer, wherein
the light-emitting layer contains at least one kind of dopant and at least one kind of host,
the light-emitting layer is formed by an application method using an application liquid containing at least one kind of solvent,
a distribution curve of a number frequency distribution of multi-molecular aggregates of either or both of molecules of the dopant and the host in the light-emitting layer has at least one peak, which distribution curve is obtained from Small-angle X-ray scattering, and
a minimum particle size at peak top(s) of the peak(s) is 1.5 nm or less.

4. An organic electroluminescent element comprising, on/over a substrate:

a pair of electrodes; and
at least one light-emitting layer, wherein
the light-emitting layer contains at least one kind of dopant and at least one kind of host,
the light-emitting layer is formed by an application method using an application liquid containing at least one kind of solvent,
a distribution curve of a number frequency distribution of multi-molecular aggregates of either or both of molecules of the dopant and the host in the light-emitting layer has at least one peak, which distribution curve is obtained from Small-angle X-ray scattering,
a minimum particle size at peak top(s) of the peak(s) is 1.5 nm or less, and
a particle size distribution of the multi-molecular aggregates also ranges over 1.5 nm to 15 nm or less.

5. The organic electroluminescent element of any one of claims 1 to 4, wherein
the distribution curve of the number frequency distribution of the multi-molecular aggregates of either or both of the molecules of the dopant and the host in the light-emitting layer has the at least one peak, which distribution curve is obtained from the Small-angle X-ray scattering,
the minimum particle size at the peak top(s) of the peak(s) is 1.5 nm or less,
the particle size distribution of the multi-molecular aggregates also ranges over 1.5 nm to 15 nm or less, and
the particle size distribution ranging over 1.5 nm to 15 nm or less accounts for 95% or less of a total.

6. The organic electroluminescent element of any one of claims 1 to 4, wherein
the distribution curve of the number frequency distribution of the multi-molecular aggregates of either or both of the molecules of the dopant and the host in the light-emitting layer has the at least one peak, which distribution curve is obtained from the Small-angle X-ray scattering,
the minimum particle size at the peak top(s) of the peak(s) is 1.5 nm or less,
the particle size distribution of the multi-molecular aggregates also ranges over 1.5 nm to 15 nm or less, and
the particle size distribution ranging over 1.5 nm to 15 nm or less accounts for 70% or less of a total.

7. The organic electroluminescent element of any one of claims 1 to 4, wherein
the distribution curve of the number frequency distribution of the multi-molecular aggregates of either or both of the molecules of the dopant and the host in the light-emitting layer has the at least one peak, which distribution curve is obtained from the Small-angle X-ray scattering,
the minimum particle size at the peak top(s) of the peak(s) is 1.5 nm or less,
the particle size distribution of the multi-molecular aggregates also ranges over 1.5 nm to 15 nm or less, and
the particle size distribution ranging over 1.5 nm to 15 nm or less accounts for 50% or less of a total.

8. The organic electroluminescent element of any one of claims 1 to 4, wherein
the distribution curve of the number frequency distribution of the multi-molecular aggregates of either or both of the molecules of the dopant and the host in the light-emitting layer has the at least one peak, which distribution curve is obtained from the Small-angle X-ray scattering,
the minimum particle size at the peak top(s) of the peak(s) is 1.5 nm or less,
the particle size distribution of the multi-molecular aggregates also ranges over 1.5 nm to 15 nm or less, and
the particle size distribution ranging over 1.5 nm to 15 nm or less accounts for 35% or less of a total.

9. The organic electroluminescent element of claim 1, wherein

a molecular weight of the host is 2000 or less.

**10.** The organic electroluminescent element of claim 1, wherein a molecular weight of the host is 800 or less.

**11.** The organic electroluminescent element of claim 1, wherein the light-emitting layer contains at least three kinds of hosts.

**12.** The organic electroluminescent element of claim 1, wherein the light-emitting layer contains at least five kinds of hosts.

**13.** The organic electroluminescent element of claim 1, wherein the light-emitting layer contains at least three kinds of dopants.

**14.** The organic electroluminescent element of claim 1, wherein the light-emitting layer contains at least five kinds of dopants.

**15.** The organic electroluminescent element of claim 1, wherein the application liquid of the light-emitting layer contains a mixed solvent composed of two or more kinds of solvents.

**16.** The organic electroluminescent element of claim 1, wherein the light-emitting layer is formed in an application environment of 35 ° C or more.

**17.** The organic electroluminescent element of claim 1, wherein the light-emitting layer is formed by drying within three seconds.

**18.** The organic electroluminescent element of claim 1, wherein the light-emitting layer contains a host material having a molecular weight of 2000 or less, and at least one of the host material(s) contains a structure represented by a following general formula (1); [Chemical Formula 1]

General formula 1

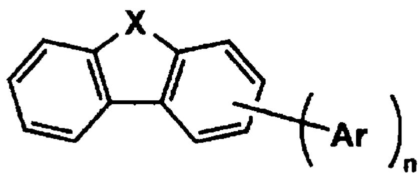

wherein in the general formula (1), "X" represents NR', O, S, CR'R" or SiR'R"; "R"' and "R"" each represent a hydrogen atom or a substituent; "Ar" represents an aromatic ring; and "n" represents an integer from 0 to 8.

**19.** The organic electroluminescent element of claim 1, wherein the dopant in the light-emitting layer is represented by a following general formula (2); [Chemical Formula 2]

General formula 2

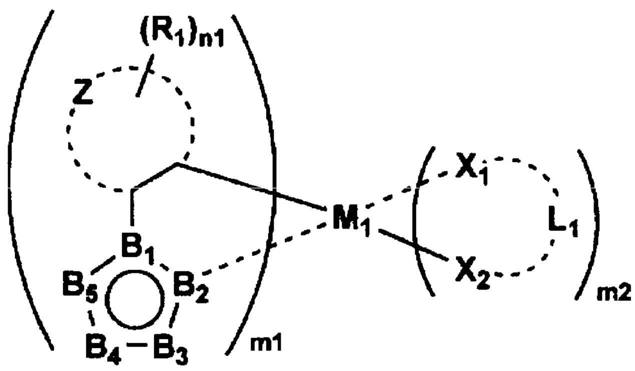

wherein in the general formula (2), "R1" represents a substituent; "Z" represents a group of non-metal atoms necessary for forming a five to seven-membered ring; "n1" represents an integer from 0 to 5; "B1 to B5" each represent a carbon atom, a nitrogen atom, an oxygen atom or a sulfur atom; at least one of "B1 to B5" represents a nitrogen atom; "M1" represents a metal of Group 8 to 10 of the periodic table; "X1" and "X2" each represent a carbon atom, a nitrogen atom or an oxygen atom; "L1" represents a group of atoms forming a bidentate ligand together with X1 and X2; "m1" represents an integer 1, 2 or 3, "m2" represents an integer 0, 1 or 2; and "m1 + m2" is equal to 2 or 3.

**20.** The organic electroluminescent element of claim 19, wherein the general formula (2) is represented by a following general formula (3);
[Chemical Formula 3]

General formula (3)

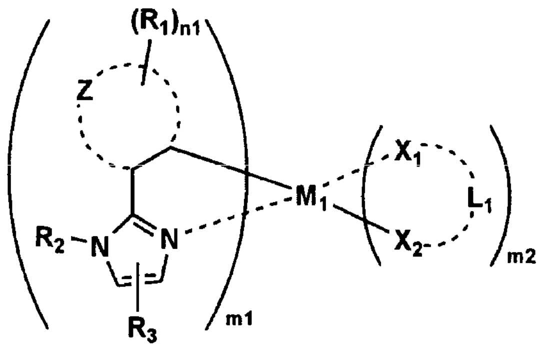

wherein in the general formula (3), "R1", "R2" and "R3" each represent a substituent; "Z" represents a group of non-metal atoms necessary for forming a five to seven-membered ring; "n1" represents an integer from 0 to 5; "M1" represents a metal of Group 8 to 10 of the periodic table; "X1" and "X2" each represent a carbon atom, a nitrogen atom or an oxygen atom; "L1" represents a group of atoms forming a bidentate ligand together with X1 and X2; "m1" represents an integer 1, 2 or 3; "m2" represents an integer 0, 1 or 2; and "m1 + m2" is equal to 2 or 3.

**21.** The organic electroluminescent element of claim 20, wherein the substituent represented by R2 in the general formula (3) is represented by a following general formula (4);
[Chemical Formula 4]

General formula (4)

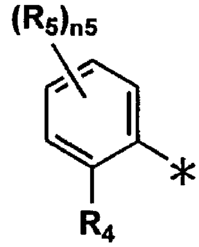

wherein in the general formula (4), "R4" represents a substituent having a steric parameter (Es value) of -0.5 or less; "R5" represents a substituent; "n5" represents an integer from 0 to 4; and "*" represents a linking position.

**22.** A display device comprising the organic electroluminescent element of claim 1.

**23.** An illumination device comprising the organic electroluminescent element of claim 1.

FIG.1

100

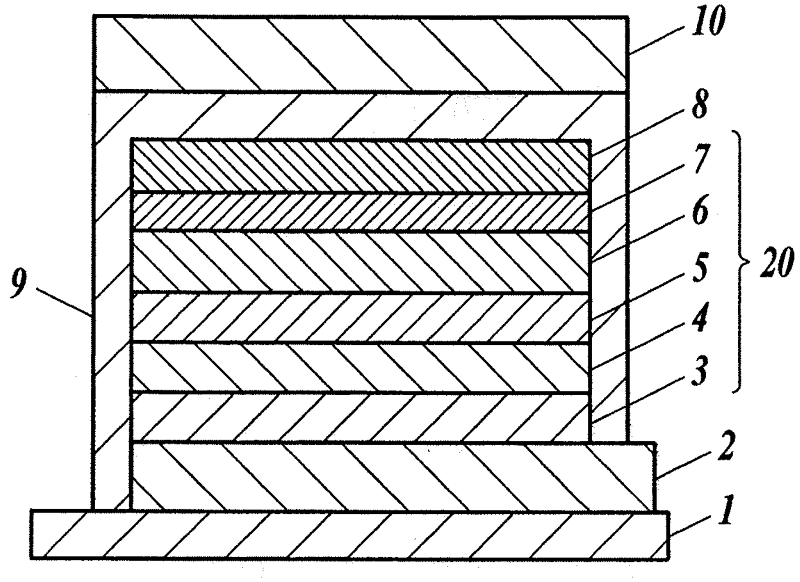

*FIG.2*

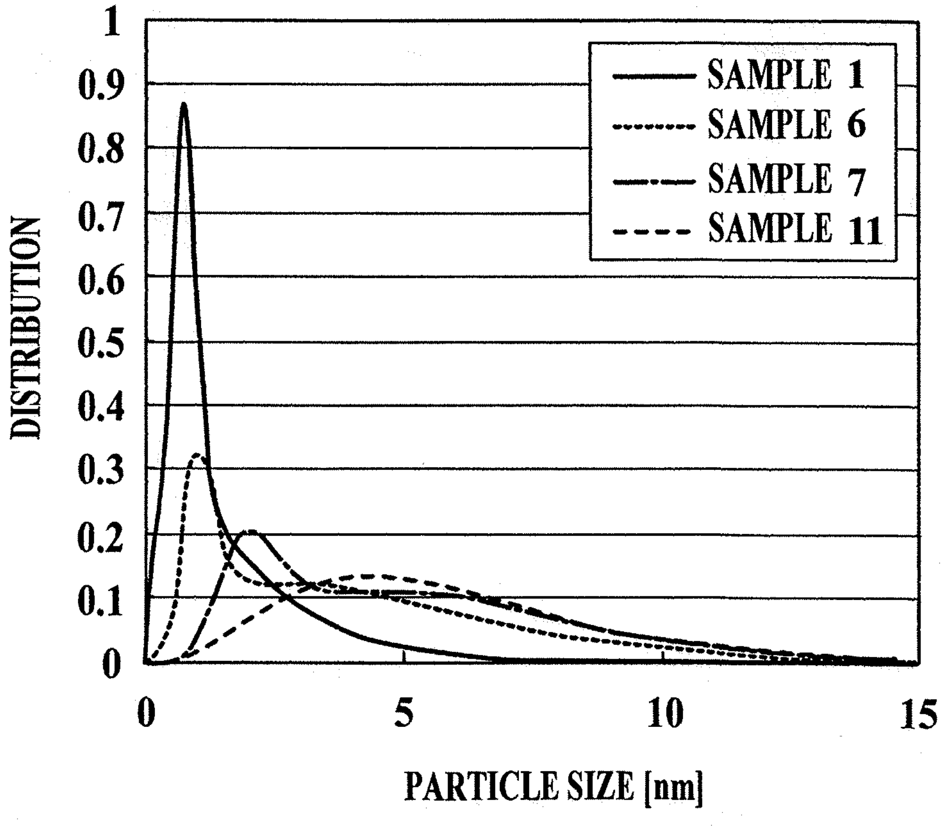

1
0.9
0.8
0.7
0.6
0.5
0.4
0.3
0.2
0.1
0
DISTRIBUTION
SAMPLE 1
SAMPLE 6
SAMPLE 7
SAMPLE 11
0
5
10
15
PARTICLE SIZE [nm]

**INTERNATIONAL SEARCH REPORT**

International application No.
PCT/JP2012/068571

A. CLASSIFICATION OF SUBJECT MATTER
*H01L51/50*(2006.01)i, *C07D519/00*(2006.01)i, *C09K11/06*(2006.01)i, *C07F15/00*(2006.01)n

According to International Patent Classification (IPC) or to both national classification and IPC

B. FIELDS SEARCHED

Minimum documentation searched (classification system followed by classification symbols)
H01L51/50, C07D519/00, C09K11/06, C07F15/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2010-278287 A (Konica Minolta Holdings, Inc.), 09 December 2010 (09.12.2010), paragraphs [0018], [0049], [0082] (Family: none) | 1-23 |
| A | JP 2010-206191 A (Mitsubishi Chemical Corp.), 16 September 2010 (16.09.2010), paragraph [0046] (Family: none) | 1-23 |
| A | WO 2010/098246 A1 (Nippon Steel Chemical Co., Ltd.), 02 September 2010 (02.09.2010), paragraphs [0008], [0009] & EP 2403028 A1 & CN 102326273 A & TW 201100518 A & KR 10-2011-0134885 A | 1-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 September, 2012 (13.09.12) | 25 September, 2012 (25.09.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/068571

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-148045 A (Samsung SDI Co., Ltd.), 08 June 2006 (08.06.2006), paragraphs [0025], [0026] & US 2006/0103298 A1 & US 2009/0197497 A1 & KR 10-2006-0055061 A & CN 1780019 A | 1-23 |
| A | JP 2003-68466 A (Canon Inc.), 07 March 2003 (07.03.2003), paragraphs [0020], [0029], [0030] & US 2003/0141809 A1 & EP 1399002 A1 & WO 2002/104080 A1 & CN 1518849 A | 1-23 |
| A | JP 2009-212510 A (Mitsubishi Chemical Corp.), 17 September 2009 (17.09.2009), paragraph [0070] (Family: none) | 1-23 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI633048 B
- JP 2006190759 A
- JP 2006066294 A
- JP 2005310639 A
- JP 2005276748 A
- JP 2010278287 A
- JP HEI945479 B
- JP 9260062 A
- JP HEI8288069 B
- JP HEI6325871 B
- JP 9017574 A
- JP HEI1074586 B
- US 5061569 A
- JP HEI4308688 B
- JP HEI4297076 B
- JP 2000196140 A
- JP 2001102175 A
- JP HEI11251067 B
- JP 2003519432 A
- JP HEI10270172 B
- JP 2001257076 A
- JP 2002308855 A
- JP 2001313179 A
- JP 2002319491 A
- JP 2001357977 A
- JP 2002334786 A
- JP 2002008860 A
- JP 2002334787 A
- JP 2002015871 A
- JP 2002334788 A
- JP 2002043056 A
- JP 2002334789 A
- JP 2002075645 A
- JP 2002338579 A
- JP 2002105445 A
- JP 2002343568 A
- JP 2002141173 A
- JP 2002352957 A
- JP 2002203683 A
- JP 2002363227 A
- JP 2002231453 A
- JP 2003003165 A
- JP 2002234888 A
- JP 2003027048 A
- JP 2002255934 A
- JP 2002260861 A
- JP 2002280183 A
- JP 2002299060 A
- JP 2002302516 A
- JP 2002305083 A
- JP 2002305084 A
- JP 2002308837 A
- JP 2004068143 A


### Non-patent literature cited in the description


- Organic EL element and its frontier of industrialization. Electrode Materials. NTS Corporation, 30 November 1998, 123-166
- *J. Appl. Phys.,* 2004, vol. 95, 5773
- **J. HUANG et al.** *Applied Physics Letters,* 2002, vol. 80, 139
- *Makromol. Chem.,* 1992, vol. 193, 909
- fourth series of Experimental Chemistry 7, Spectroscopy II. MARUZEN Co., Ltd, 1992, 398